(19) Europäisches Patentamt
European Patent Office
Office européen des brevets

(11) Publication number: **0 369 409 B1**

# (12) EUROPEAN PATENT SPECIFICATION

(45) Date of publication of patent specification: **04.01.95**

(51) Int. Cl.[6]: **C07F 9/6512**, A61K 31/675, C07F 9/6561

(21) Application number: **89121085.8**

(22) Date of filing: **14.11.89**

## (54) Carbocyclic nucleosides and nucleotides.

(30) Priority: **14.11.88 US 270331**

(43) Date of publication of application: **23.05.90 Bulletin 90/21**

(45) Publication of the grant of the patent: **04.01.95 Bulletin 95/01**

(84) Designated Contracting States: **AT BE CH DE ES FR GB GR IT LI LU NL SE**

(56) References cited:
**EP-A- 0 015 584**

**COLLECTION OF CZECHOSLOVAK CHEMICAL COMMUNICATIONS, vol. 52, no. 10, October1987, pages 2572-2588, Academia Nakladatelstvi Ceskoslovenské Akademie Ved; I.ROSENBERG et al.: "Synthesis of phosphonylmethyl analogues of diribonucleosidemonophosphates containing modified internucleotide bond"**

(73) Proprietor: **INSTITUTE OF ORGANIC CHEMISTRY AND BIOCHEMISTRY OF THE ACADEMY OF SCIENCES OF THE CZECH REPUBLIC**
**Flemingovo namesti 2**
**CS-166 10 Praha 6 (CS)**

Proprietor: **Rega Stichting Vzw.**
**Minderbroedersstraat 10**
**B- 3000 Leuven (BE)**

(72) Inventor: **Mansuri, Muzammil M.**
**1320 Deer Run Road**
**Cheshire**
**Connecticut 06410 (US)**
Inventor: **Martin, John C.**
**40 Brookside Place**
**Cheshire**
**Connecticut 06416 (US)**
Inventor: **Hudyma, Thomas W.**
**P.O. Box 245**
**Durham**
**Connecticut 06422 (US)**

(74) Representative: **Kinzebach, Werner, Dr. et al**
**Patentanwälte**
**Reitstötter, Kinzebach und Partner**
**Postfach 86 06 49**
**D-81633 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to carbocyclic purine and pyrimidine nucleoside analogs having a cyclopentane or cyclopentene ring instead of the deoxyribofuranose glycosidic substituent of the natural nucleosides (Class 514, Subclasses 262 and 269). The compounds are further characterized in that they have carbon-attached phosphorous-containing substituents (Class 514, Subclass 86).

The carbocyclic analog of thymidine has been described by Shealy, et al. in J. Heterocyc. Chem 13, 1041-1047 (1976).

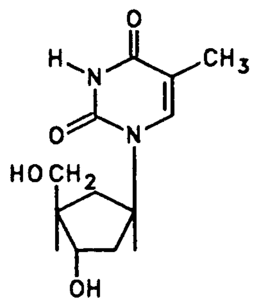

Other references disclosing purine and pyrimidine bases having respectively an $N^{9-}$, or $N^{1-}$ cyclopentyl substituent of this type are disclosed in the following references.

Shealy, et al., J. Heterocyc. Chem. 18, 383-389, (1981).

Shealy, et al., J. Med. Chem. 26, 156-161 (1983).

Taniyama, et al., European Specification 236,935 published Sept. 16, 1987.

Shealy, et al., U. S. Patent No. 4,730,001 patented March 8, 1988.

Shealy, et al., U. S. Patent No. 4,396,623 patented August , 1983.

Shealy, et al., U. S. Patent NO. 4,719,214 patented Jan. 12, 1988.

The foregoing references disclose antiviral activity.

The neplanocins are antitumor antibiotics in which the ribose unit of a purine nucleoside is replaced by a substituted cyclopentene ring. Five of them have been isolated from Ampullarilla regularis A11079 fermentationm broths. Their structures have been determined, and they have been synthesized (Hayashi, et al., J. Antibiot. 34, 675-680, (1981), Lim, et al., Tetrahedron Lett. 24. 5559-5562 (1983).

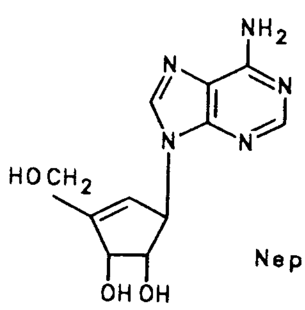

Neplanocin A

Of significance to the present invention is the synthesis of the antibiotic aristeromycin by Trost, et al., J. Am. Chem. Soc.(1988), 110, 621-622 by the following route.

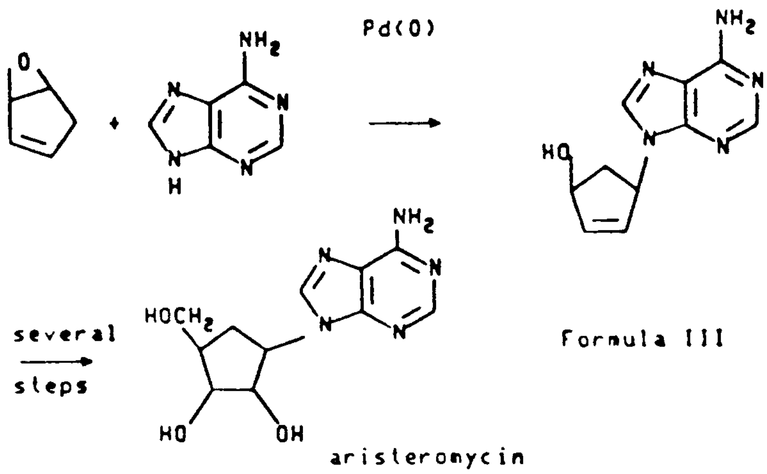


Formula III

aristeromycin

Aristeromycin is a fermentation product having growth inhibitory activity against phytopathogenic bacteria and fungi (Kusaka, et al., J.Antibiotics, (1968), 21, 255). It is cytotoxic, active against murine leukemia L 1210 in vitro, and has antiviral activity (Herdewijn, et al., J. Med. Chem. (1985), 28, 1385-1386).

A series of 3-heterocyclo-5-hydroxymethylcyclopentanols having strong antiviral activity against Herpes simplex virus 2, strain G is disclosed in U. S. Patent No. 4,605,659 of Verheyden, et al., patented Aug. 12, 1986.

Another group of antiviral nucleoside compounds is typified by the phosphonomethoxyadenine derivatives typified by (S)-HPMPA which has the following formula (Holy, et al., Nucleic Acids Research, Symposium Series No. 14, 1984, pages 277-278, and UK specification 2,134,907, published Aug. 22, 1984).

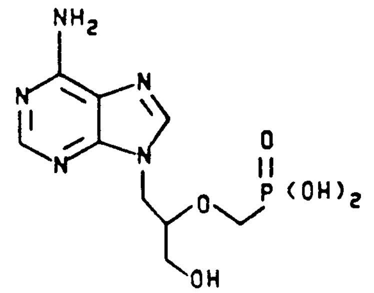

(S) - HPMPA

This invention refers to compounds of Formulas I and II, to methods for their synthesis, to the use thereof for treating infections of viral or microbial origin in animals or plants and to compositions useful for the latter purpose.

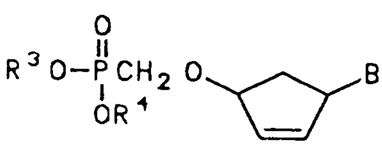

I

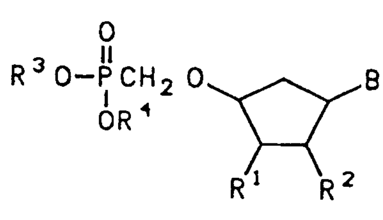

II

In Formulas I and II, the symbols B, $R^1$, $R^2$, $R^3$, and $R^4$, have the following meanings. $R^1$ and $R^2$ are independently hydrogen, hydroxy, chlorine, bromine, amino, or an organic substituent selected from acyloxy, alkoxy, alkylthio, alkylamino, and dialkylamino. The latter contain from 1 to 12 carbon atoms. $R^3$ and $R^4$ are independently hydrogen, or an organic substituent having 1 to 12 carbon atoms and selected

from alkyl, alkenyl, aryl, and aralkyl. B is a heterocyclic group having at least one nitrogen heteroatom and up to three additional heteroatoms selected from nitrogen, oxygen and sulfur, said heterocyclic group being connected through a nitrogen heteroatom thereof, and the metal and amine salts of those compounds of Formulas I and II wherein at least one of $R^3$ and $R^4$ is hydrogen.

B is preferably selected from purine, pyrimidine, azapurine, triazine, deazapurine, pyridine, and triazole, said heterocyclic group being substituted with from 1 to 3 substituents independently selected from oxo, hydroxy, amino, fluoro, chloro, bromo, iodo, alkyl having 1 to 3 carbon atoms, alkenyl having 2 to 3 carbon atoms, haloalkenyl having 2 to 3 carbon atoms, alkoxy having 1 to 3 carbon atoms, and alkylthiol having 1 to 3 carbon atoms and in particular monosubstituted purine, disubstituted purine, monosubstituted pyrimidine, disubstituted pyrimidine or trisubstituted pyrimidine.

The compounds of the present invention preferably have the 1β, 4β-conformation.

Particularly preferred compounds are:

1-[(1β,4β)-4-(dihydroxyphosphonyl)methoxycyclopent-2-en-1-yl]thymine,
1-[(1β,4β)-4-(dihydroxyphosphonyl)methoxycyclopent-2-en-1-yl]cytosine,
[(1β,4β)-4-(diethoxyphosphonyl)methoxycyclopent-2-en-1-yl]adenine,
9-[(1β,4β)-4-(ethoxyhydroxyphosphonyl)methoxycyclopent-2-en-1-yl]adenine,
9-[(1β,4β)-4-(dihydroxyphosphonyl)methoxycyclopent-2-en-1-yl]adenine,
9-[(1β,4β)-4-(diethoxyphosphonyl)methoxycyclopentan-1-yl]adenine,
9-[(1β,4β)-4-(dihydroxyphosphonyl)methoxycyclopentan-1-yl]adenine.

Preferred embodiments are the compounds of the following formulae:

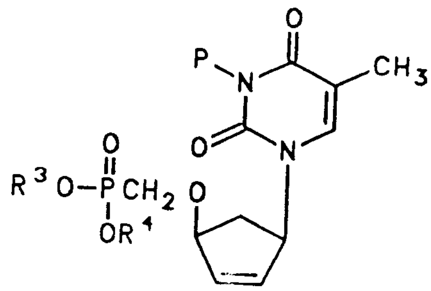

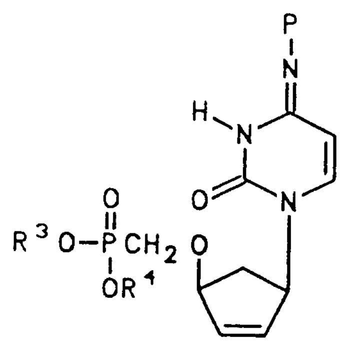

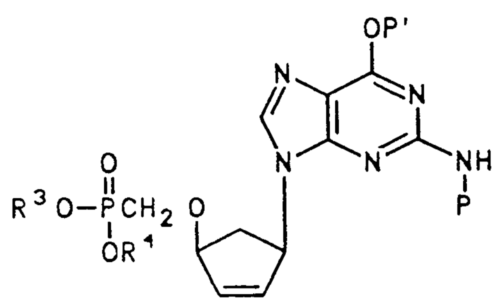

wherein $R^3$ and $R^4$ have the definitions given above, P is hydrogen or an amino protecting group and P' is hydrogen or a hydroxy protecting group. Formulas I and II are shown in a non-specific representation with respect to stereochemical configuration but only the cis stereochemical form relative to B and the phosphonylmethoxy group in the 4-position is intended.

The compounds of the invention are used to treat viral diseases and diseases of microbial origin in animals (including man) or plants. The invention includes compositions useful for these purposes. They are also effective against infectious conditions caused by other microorganisms, and against tumors in experimental animals.

The salts just alluded to are considered part of the present invention. Those salts which are pharmaceutically acceptable are of particular interest since they are useful in administering the foregoing compounds for medical purposes. Some salts which are not pharmaceutically acceptable are useful in manufacturing processes, for isolation and purification purposes, and in some instances, for use in separating stereoisomeric forms of the compounds of Formulas I and II. The latter is particularly true of amine salts prepared from optically active amines.

Pharmaceutically acceptable metal and amine salts are those salts which are stable under ambient conditions, and wherein the cation does not contribute significantly to the toxicity or biological activity of the salt. Suitable metal salts include the sodium, potassium, calcium, barium, zinc, and aluminium salts. The sodium and potassium salts are preferred. Suitable amine salts are prepared from amines which have sufficient basicity to form a stable salt, and preferably include those amines which are frequently used in medicinal chemistry because of their low toxicity and acceptability for medical use. These include the trialkylamines such as triethylamine, and others including procaine, dibenzylamine, N-benzyl-beta-phenethylamine, ephenamine, and N,N′-dibenzylethylenediamine, dehydroabietylamine, N-ethylpiperidine, benzylamine, and dicyclohexylamine.

Those compounds of Formulas I and II in which $R^1$ or $R^2$ is a basic function such as the amino, alkylamino, or dialkylamino group or if such group is present as a substituent on B, $R^1$, or $R^2$, will form acid addition salts. Again, such salts are intended to be included in the present invention. As before, the pharmaceutically acceptable acid addition salts are preferred. They are the acid addition salts in which the anion does not contribute significantly to the toxicity of the salt, and which salts are compatible with the customary pharmaceutical vehicles and adapted for oral or parenteral administration to animals or for application to plants. Some suitable acids for use in the preparation of such salts are hydrochloric acid, hydrobromic acid, phosphoric acid, sulfuric acid, various organic carboxylic and sulfonic acids such as acetic acid, citric acid, propionic acid, succinic acid, benzoic acid, tartaric acid, fumaric acid, mandelic acid, ascorbic acid, malic acid, methanesulfonic acid, toluenesulfonic acid, and others.

## DOSAGE AND ADMINISTRATION

The present substances have utility as antiviral, antimicrobial, and antitumor agents. Their antiviral properties can be measured and demonstrated by any of a variety of laboratory methods which are available for this purpose. Similarly, utility against plant pathogens and microbial infections caused by bacteria and other microorganisms can be demonstrated by methods which are well known to those skilled in the art. Their antitumor activity can be shown by established techniques in animals bearing experimental tumors or *in vitro* by using various animal and human tumors. The particular spectrum of viruses and microorganisms against which the present substances are active varies from compound to compound. The murine leukemia virus is an example of a retrovirus which is sensitive to the present compounds. A number of disease conditions including AIDS are caused by retroviruses. The test method is given below and results for several compounds are presented in Table I.

## TESTING AND EVALUATING OF COMPOUNDS AGAINST MURINE RETROVIRUSES.

The compounds were evaluated for antiviral activity against Murine leukemia virus (MuLV) strains using the UV-XC plaque assay (Rowe, et al., "Virology," 42:1136, 1970).

The MuLV strains were grown in feral mouse cells (SC-1) and used for antiviral tests using the UV-XC plaque assay. Briefly, SC-1 cells are grown as monolayers in 4-well tissue culture plates and inoculated with approximately 50-100 plaque forming units of MuLV in 0.5 ml of 5% EMEM (Earle's Minimum Essential Medium) containing 20 mcg/ml-DEAE/Dextran. After 1 hr. adsorbtion, the inoculum is removed and 5 ml of 5% EMEM containing three-fold dilutions of the appropriate drug are added. Five days later, the cultures are UV irradiated with an ultraviolet lamp and rat XC sarcoma cells are added to the cultures. Three or four days after UV-irradiation, the cell cultures are stained with Giemsa stain and the plaques are counted. Antiviral activity is expressed in terms of the reduction in the mean number of UV-XC plaques counted in the drug treated, virus-infected cultures compared with mean number of plaques counted in untreated, virus-infected control cultures, and reported as $ID_{50}$ (mcg/ml). The $ID_{50}$ is the concentration of test substance in the medium which reduces the number of plaques formed by 50%. It is usually determined by interpolation from a concentration response graph prepared after testing several multiple concentrations.

Table I

In Vitro Murine Leukemia Virus $ID_{50}$

| Compound No.[2] | Formula | Structure[1] $R^3$ | $R^4$ | Virus | toxicity (mcg/ml) | $ID_{50}$ (mcg/ml) | Index |
|---|---|---|---|---|---|---|---|
| AZT[3] | not applicable | | | Maloney | - | 0.001 | - |
| 40445 | I | $C_2H_5$ | $C_2H_5$ | Maloney | 32 | 4.68 | 6.8 |
| 40803 | I | H | H | Maloney | >32 | >1 | >32 |
| 40815 | II | $C_2H_5$ | $C_2H_5$ | Maloney | 32 | 7.69 | 4.2 |
| 40815 | II | $C_2H_5$ | $C_2H_5$ | Rauscher | >32 | 10.5 | >3.0 |
| 40843 | II | H | H | Maloney | >32 | 4.88 | >6.6 |
| 40843 | II | H | H | Maloney | >32 | 3.29 | >9.7 |

[3] AZT is a commercial anti-viral agent, non-proprietary name zidovudine, 3'-azido-3'-deoxythymidine.

[1] B in Formula I and Formula II is 9-adeninyl; $R^1$ and $R^2$ of Formula II are each H.

[2] Preparative procedures are found in the examples numbered as follows:

| | |
|---|---|
| 40445 | Example V |
| 40803 | Example EE |
| 40815 | Example FF |
| 40843 | Example GG |

Pharmaceutical compositions, both veterinary and human, containing the claimed compounds which are appropriate for antiviral use are prepared by methods and contain excipients which are well known in the art. A generally recognized compendium of such methods and ingredients is Remington's Pharmaceutical Sciences by E. W. Martin, (Mark Publ. Co.,15th Ed., 1975).

The compounds of the invention may be administered parenterally (for example, by intravenous, subcutaneous, intraperitoneal, or intramuscular injection), orally, topically, intranasally, or rectally.

The compositions are administered orally or parenterally at dose levels of about 0.1 to 300 mg/kg of compound of Formula I or compound of Formula II, preferably 1.0 to 30 mg/kg of body weight. Unit dosage forms administered one to five times daily in the amount of 10 to 500 mg per unit dose, are contemplated for man.

For parenteral administration or for administration as drops, as for eye infections, the compounds may be presented in aqueous solution in a concentration of from about 0.1 to 10%, more preferably about 0.1 to 7%. The solution may contain antioxidants, buffers, and other suitable additives.

Alternatively for infections of the eye, or other external tissues, e.g. mouth and skin, the compositions are preferably applied to the infected part of the body of the patient topically as an ointment, cream, aerosol or powder, preferably as an ointment or cream. The compounds may be presented in an ointment, for instance with a water soluble ointment base, or in a cream, for instance with an oil in water cream base, in a concentration of from about 0.01 to 10%.

The compounds of the present invention or compositions containing them are also useful in treating non-human mammals, bird, eg., chickens and turkeys, and cold-blooded animals, e.g., fish.

Fish which are in a confined area such as a pool, aquarium, or holding tank may also be treated for viral infections such as herpes-like viruses, e.g., channel catfish virus (CCV), herpes-virus salomones, Nerka virus and the like by adding the compound directly to the water of the pool, aquarium, or holding tank or by incorporating the compounds into the feed.

The exact regimen for administration of the compounds and compositions disclosed herein will necessarily be dependent upon the needs of the individual subject being treated, the type of treatment and, of course, the judgment of the attending physician.

## PREPARATIVE METHODS

The phosphonic esters of Formula I wherein $R^3$ and $R^4$ as defined, but other than hydrogen, are useful end products and also organic phosphonic ester substituents are key intermediates for the preparation of the other compounds of the present invention. Considering the cyclopentene ring as the central nucleus of these compounds they are prepared by either of two sequences. According to the preferred sequence the purine or pyrimidine base substituent B is first introduced to provide a cis-4-hydroxycyclopent-2-en-1-yl derivative of Formula IV. The cyclopentenol of Formula IV is then etherified by treatment with a phosphonyl methylating agent of Formula V in which L is a leaving group. The preferred phosphonylmethylating agent is diethyl phosphonylmethyltrifluoromethane sulfonate (Kluge, Organic Synthesis (1985), 64 80). When operating on a pyrimidine base, the use of protecting groups for the ring nitrogen atom in the 3 position or 4-amino substituent when present is sometimes desirable. Whether or not this expedient is required in a given instance is readily determined by the skilled chemist by carrying out trial reactions on a small scale.

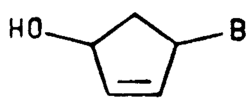

IV

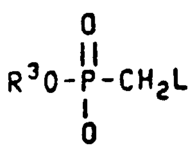

V

Alternatively the phosphonylmethyl ether group may be introduced first to provide a 1,4 disubstituted cyclopent-2-ene of Formula VI, where L is a leaving group in the trans-configuration.

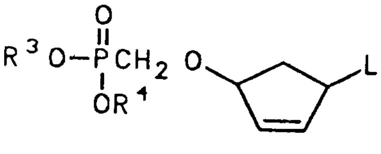

VI

Again, the leaving group L is of a similar nature as with respect to Formula V and may include halogen, alkylsulfonate or arylsulfinate.

The method of Trost, et al. cited above, J. Med. Chem. Soc. (1988), 110, 621-622 involving reaction of adenine with 3,4-epoxycyclopent-2-ene under the control of palladium (0) has proven to be suitable for the preparation of the Formula IV intermediates for the preferred sequence outlined above. The reaction proceeds in good yield, and produces the desired 1$\beta$,4$\beta$ cyclopent-2-ene nucleoside analog stereoselectively. The intermediates of Formula VI required for the alternate route may be prepared from 3,4-epoxycyclopent-2-ene which on reaction with thiophenol in the presence of triethylamine (D. A. Evans, et al., J. Org. Chem. 1976, 39, 3178) yields trans-1-hydroxy-2-phenylthiocyclopent-3-ene. This may be etherified with dietoxyphosphonylmethyl trifluoromethanesulfonate as described above. Oxidation of the phenyl sulfide to the sulfoxide and rearrangement and hydrolysis yields the monoetherified transcyclopent-2-ene-1,4-diol of Formula VII

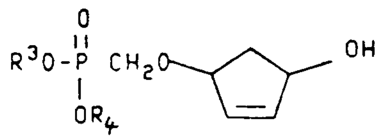

Formula VII

The leaving group L of Formula VI is conveniently an alkyl-or arylsulfinate obtained by reaction of the compound of Formula VII with phenylsulfiyl chloride (or other arylsulfinyl chloride) or an alkylsulfinyl chloride. The alkylsulfinates or arylsulfinates so produced are then converted to the compounds of Formula I by suitable displacement reactions. Those compounds of Formula VI wherein L is chlorine can be heated with the sodium salt form of the chosen heterocycle B in the presence of sodium bromide or sodium iodide to yield the desired cis-1,4-substituted products of Formula I.

According to a further alternate method the monoetherified trans-dial of Formula VII may be converted directly to products of Formula I by means of a Mitsunabu reaction (O. Mitsanobu, Synthesis 1981, 1) in which the heterocyclic base B acts as the nucleophile.

The compound of Formula I is then transformed into a compound of Formula II according to any of a number of well established techniques in organic chemistry. For example, catalytic hydrogenation of a compound of Formula I yields a compound of Formula II in which $R^1$ and $R^2$ are hydrogen atoms. Another technique that may be used is hydroxylation. Hydroxylation with osmium tetroxide and N-methylmorpholine-N-oxide according to Van Rheenan, et al., Tetrahedron Letters. 1976, 1973 yield the dehydroxy compound ($R^1$ and $R^2$ are each OH). Hydroboration yields a mixture of monohydroxy compounds which can be separated (one of $R^1$ and $R^2$ is OH and the other is H). The resulting hydroxy compounds can then be converted to various derivatives of the hydroxyl group such as the acyloxy, alkoxy, alkiothio, halogen, amino, alkylamino and dialkylamino groups be methods available in the art.

The di-esters of Formula I and Formula II may be hydrolyzed to the corresponding monoesters, wherein one of $R^3$ and $R^4$ is a hydrogen atom and the other is an organic phosphonic ester substituent as before. Hydrolysis may be carried out with aqueous sodium hydroxide solution at room temperature to yeild the monoester. The dibasic acid of Formula I or Formula II is prepared by cleavage of the corresponding mono or diester with trimethylsilyl bromide. This reaction is carried out in the absence of water using dimethylformamide or aceto nitrile as solvent. Room temperature and protection of the reaction mixture from the atomosphere are preferred conditions.

Description of Specific Embodiments

Abbreviations Used

| | |
|---|---|
| THF | tetrahydrofuran |
| SEM-Cl | 2-(trimethylsilyl)ethoxymethyl chloride |
| HPLC | high performance liquid chromatography |
| rt | room temperature |
| HOAc | acetic acid |
| mCPBA | m-chloroperbenzoic acid |
| DMF | dimethylformamide |
| IPA | isopropyl alcohol |
| EtOAc | ethyl acetate |

**EXAMPLE A: (±)-9-(4-β-Hydroxycyclopent-2-ene-1-β-yl)adenine**

A solution of 3,4-epoxycyclopentene (194 mg, 2.36 mmol) in DMF (1.5 mL) was added dropwise over two mins to a stirred mixture of adenine (319 mg, 2.36 mmol) and tetrakis(triphenylphosphine)palladium (0) (137 mg,0.118 mmol) in DMF (5 mL) and THF (2 mL) at 22 °C under argon. There was a mild exotherm after which the reaction was stirred for 1 hr at ambient temperatures and then for 2 hrs at an oil bath temperature of 80-90 °C. The mixture was then poured into warm water and filtered. After an additional filtration through a 0.45μm nylon membrane filter the filtrate was pumped onto a Michel-Miller (310 x 25 mm) column which packed with Partisil Prep 40TM ODS-3. The column was eluted with 0.025 M of pH5 ammonium phosphate buffer containing 4-10% $CH_3CN$. The progress of elution was monitored with a refractive index detector and the appropriate fractions were combined. The pH of the combined eluates was adjusted to 7.06 with dilute NaOH. The resulting solution was concentrated and the residue was dissolved in $H_2O$ (20 mL). The solution was applied to the Michel-Miller column and the column eluted with $H_2O$ (200 mL) to remove the inorganic salts. Elution with $H_2O$-10% $CH_3CN$ afforded the title compound, which was isolated as a isolated as a colorless powder (170 mg, 33%) after removal of the $CH_3CN$ followed by lyophilization of the resulting aqueous solution. Analysis; $C_{10}H_{11}N_5O$ 0.25$H_2O$: (calc) C: 54.17, H: 5.02, N: 31.59, $H_2O$, 2.03; (found) C: 54.12, H: 5.27, N: 31.07, $H_2O$, 1.77. $^1H$ NMR (360 MHz, $D_2O$) δ 8.03 (s, 1H), 8.00 (s, 1H), 6.25 (m, 1H), 6.06 (m, 1H), 5.36 (m, 1H), 4.85 (m, 1H under HDO resonance), 3.01 ( m, 1H), 1.71 (m, 1H).

**EXAMPLE B: ( )-1-(4-β-Hydroxycyclopent-2-ene-1-β-yl)thymine**

A solution of 3,4-epoxycyclopentene (361 mg, 4.39 mmol) in DMF (1 mL) was rapidly added to a stirred, deoxygenated mixture of thymine (462 mg, 3.66 mmol) and tetrakis(triphenylphosine)palladium (0) (212 mg, 0.183 mmol) in a mixture of DMF (5 mL) and THF (2 mL) at 22 °C under argon. THe reaction mixture was then stirred at 90 °C for 1 hr. The mixture was cooled and filtered. The filtrate was diluted with $H_2O$ and concentrated to a volume of about 20 mL. The hazy solution was refiltered (0.45 μm membrane) and chromatographed and desalted on the Michel-Miller C18 column as described in the previous example to provide an aqueous solution of the title compound. The solution was concentrated on a rotary evaporator to a volume of about 5 mL, whereupon colorless crystals (121 mg, 16%) of the analytical sample were formed. M.p. 197 - 198 °C.

Analysis: $C_{10}H_{12}N_2O_3$ (calc.) C: 57.69, H: 5.81, N: 13.45; (found) C: 57.39, H: 5.90, N: 13.38. $^1H$ NMR (360 MHz, $D_2O$) δ 7.38 (s, 1H), 6.20 (m, 1H), 5.88 (m, 1H), 5.41 (m, 1H), 4.83 (m, 1H), 2.94 (m, 1H), 1.84 (s, 3H), 1.45 (m, 1H).

(±)-1-(4-β-Hydroxycyclopent-2-ene-1-β-yl)thymine was isolated in a comparable yield when the tetrakis-(triphenylphosphine) palladium (0) catalyst was replaced with $Pd[P(i\text{-}OC_3H_7)_3]_4$ as the palladium (0) catalyst in the above experiment.

The isolated yield of (±)-1-(4-β-Hydroxy-2-cyclopenten-1-β-yl)thymine was 36% when a solution of 3,4-epoxycyclopentene (4.61 g, 56.1 mmol) in DMF (7 mL) was added dropwise over 40 minutes to a stirred, deoxygenated mixture of thymine (4.4g, 34.6 mmol) and tetrakis(triphenylphosine)palladium (0) (2.0 g, 1.73 mmol) in DMF (60 mL) at an oil bath temperature of 90 °C. Stirring was continued at 90 °C for 3.25 hrs and the title compound isolated and purified by chromatography on a Michel-Miller column (40x350mm) as previously described.

**EXAMPLE C: (±)-1-(4-β-Hydroxycyclopent-2-ene-1-β-yl) cytosine.**

A solution of 3,4-epoxycyclopentene (272 mg, 3.31 mmol) in DMF (1 mL) was rapidly added to a stirred, deoxygenated mixture of cytosine (307 mg, 2.76 mmol) and tetrakis(triphenylphosphine)palladium(0) (160 mg, 0.138 mmol) in THF (2 mL) and DMF (5 mL) at 22 °C. An immediate mild exothermic reaction ensued. THe reaction mixture was stirred for 72 hrs at ambient temperature. The mixture was filtered and the filtrate was diluted with $H_2O$. The hazy filtrate was refiltered (0.45mm nylon membrane) and the clarified solution pumped onto the Michel-Miller (310 x 25 mm) $C_{18}$ column. The column was eluted with 0.025 M of pH 4.8 ammonium phosphate buffer containing 5% $CH_3CN$, and the progress of elution monitored by differential refractometry. The pH of the appropriately combined eluates was adjusted to 7.2 with dilute $NH_4OH$. The volume of the combined eluates was reduced to about 4 mL on a rotary evaporator. The solution was applied to the Michel-Miller $C_{18}$ column. The column was eluted with $H_2O$ to remove the inorganic material and then with $H_2O$-10% $CH_3CN$ to elute the title compound as a colorless solid (54 mg, 10.1 %) which was isolated by lyophilization. Analysis: $C_9H_{11}N_3O_2$ (calc) C: 55.98, H: 5.74, N: 21.75;

(found) C: 55.25, H: 5.87, N: 21.48. $^1$H NMR (360 MHz, $D_2O$) δ 7.53 (d, 1H), 6.20 (m, 1H), 6.00 (d,1 H), 5.90 (m, 1H), 5.41 ( m, 1H), 4.79 (m, 1H under HDO resonance), 2.96 (m, 1H), 1.41 (m, 1H).

In an improved procedure, (±)-1-(4-β-hydroxycyclopent-2-ene-1-β-yl)cytosine was isolated in a lyophilized yield of 54% when a solution of 3,4-epoxycyclopentene (790 mg, 9.62 mmol) in DMF (1.5 mL) was added dropwise over 1.25 hrs to a stirred, deoxygenated mixture of cytosine (754 mg, 6.78 mmol), tetrakis-(triphenylphosphine) palladium (0) (413 mg, 0.357 mmol) and triphenylphosphine (187 mg, 0.714 mmol) in DMF (15mL) at 22°C. The mixture was then stirred at 22°C for 0.66 hrs, 50°C for 0.6 hrs and then at 80°C for 0.25 hrs, the title compound was isolated as described above.

**EXAMPLE D: (±)-1-(4-β-Hydroxycyclopent-2-ene-1-β)-yl-N3-(2-(trimethylsilyl)ethoxymethyl)thymine**.

Method 1.

A mineral oil dispersion of 50% NaH (18 mg, 0.378 mmol) was added to a stirred mixture of (±)-1-(4-β-hydroxycyclopent-2-ene-1-β-yl)thymine (75 mg, 0.36 mmol) in DMF (0.5 mL) at 22 °C. After the rapid evolution of hydrogen subsided, the mixture was stirred at 60 °C for 5 mins to complete salt formation. The mixture was cooled in a IPA/$CO_2$ bath to -40°C to -30°C, a solution of 2-(trimethylsilyl)ethoxymethyl chloride (SEM-Cl) (61 mg, 0.36 mmol) in DMF (0.5 mL) was then added dropwise over 8 mins. Stirring was continued with cooling for 0.25 hrs and then at 22°C for 0.66 hrs. The mixture was concentrated and the residue partitioned between EtOAc and $H_2O$. The EtOAc layer was washed ($H_2O$, brine), dried ($Na_2SO_4$) and concentrated. The residue was chromatographed on $SiO_2$ (10 g) with $CH_2Cl_2$-acetone (20:3) to afford the title compound (65mg, 53% yield) as a viscous oil with an estimated purity of 93.4 % by HPLC.
HPLC: rt 5.88 mins (Waters $C_{18}$ radial pak cartridge) flow rate 2mL/min of 45% pump A (90%$H_2O$-10%$CH_3CN$), 55% pump B (20%$H_2O$-80%$CH_3CN$). Detection at 254 nm. $^1$H NMR (200 MHz, $CDCl_3$) δ 7.16 (s, 1H), 6.20 (m, 1H), 5.82 (m, 1H), 5.52 (m, 1H), 5.41 (s, 2H), 4.86 (m, 1H), 3.69 (m, 2H), 2.90 (m, 1H), 2.70 (bs, 1H), 1.92 (s, 3H), 1.61 (m, 1H), 0.98 (m, 2H), 0.02 (s, 9H).

Method 2.

A solution of (±)-1-(4-β-acetoxycyclopent-2-en-1-β-yl)-$N^3$-(2-(trimethylsilyl)ethoxymethyl)thymine (2.26 g, 5.94 mmol) in $CH_3OH$ (75 mL) was saturated with $NH_3$ at 10 °C. The solution was allowed to stand for 20 hrs at ambient temperatures and then was concentrated. The residue was partitioned between $Et_2O$ and $H_2O$. The ethereal layer was washed with $H_2O$, followed by brine and then dried over $Na_2SO_4$. Removal of the ether afforded the title compound (2.0 g, 99.5%) which was identical to that prepared in Method 1.

**EXAMPLE E: (±)-1-(4-β-Acetoxycyclopent-2-ene-1-β-yl)-N3-(2-(trimethylsilyl)ethoxymethyl)thymine and (±)-1-(4-β-Hydroxycyclopent-2-ene-1-β-yl)-N3 (2-(trimethylsilyl)ethoxymethyl)thymine.**

A mineral oil dispersion of 50% NaH (0.608 g, 12.7 mmol) was added to a cooled (ice/$H_2O$ bath), stirred mixture of (±)-1-(4-β-hydroxycyclopent-2-ene-1-β-yl)thymine (2.51 g, 12.1 mmol) in DMF (17 mL) under argon. The cooling bath was removed after the vigorous evolution of hydrogen subsided and stirring was continued at ambient temperatures for 0.25 hrs, and then for an additional 0.25 hrs at 50 °C on a steam bath. The stirred mixture was cooled in a IPA/$CO_2$ bath which was maintained at -40 °C. A solution of SEM-Cl (2.01 g, 12.1 mmol) in DMF (17 mL) was added dropwise during 5 mins. Stirring was continued with cooling for 0.25 hrs at -40 °C and then 0.5 hrs at ambient temperatures. The mixture was concentrated and the residue partitioned between EtOAc and $H_2O$. The EtOAc layer was washed ($H_2O$, brine), dried ($Na_2SO_4$) and concentrated. The residue was flash chromatographed on $SiO_2$ (150 g) with $CH_2Cl_2$-acetone (100:15) to provide (±)-1-(4-β-hydroxycyclopent-2-ene-1-β-yl)-N3-(2-(trimethylsilyl)ethoxymethyl)thymine (790 mg, 19% yield) as a viscous oil and (±)-1-(4-β-acetoxycyclopen-2-ene-1-β-yl)-N3-(2-(trimethylsilyl)ethoxymethyl)-thymine (3.1 g, 68%). Recrystallization of the latter compound from hexane afforded colorless crystals of the analytical sample. M.p. 65-66 °C. Analysis $C_{18}H_{28}N_2O_5Si$ (calc) C: 56.82, H: 7.42, N: 7.37; (found) C: 56.59, H: 7.42, N ; 7.29.
$^1$H NMR (200 MHz, $CDCl_3$) δ 7.00 (s, 1H), 6.23 (m, 1H) 5.93 (m, 1H), 5.70 (m, 2H), 5.38 (s, 2H), 3.70 (m, 2H), 3.00 m, 1H), 2.09 (s, 3H), 1.94 (s, 3H), 1.65 (m, 1H), 0.99 (m, 2H), 0.01 (s, 9H).

**EXAMPLE F: (±)-1-(4-β-Diethylphosphonylmethoxycyclopent-2-ene-1-β-yl)-N3-(2-(trimethylsilyl)-ethoxymethyl) thymine.**

Method 1.

A mineral oil dispersion of 50% NaH (10.4 mg, 0.217 mmol) was added to a stirred solution of (±)-1-(4-β-hydroxycyclopent-2-ene-1-β-yl)-N3-(2-(trimethylsilyl)ethoxymethyl)thymine(70 mg, 0.207 mmol) in DMF (0.5 mL) at 22 °C. Stirring was continued for 0.5 hrs at 22 °C and then for 0.25 hrs at 60 °C. The solution was cooled in an IPA/$CO_2$ bath maintained at -40 °C when a solution of diethyl phosphonomethyltrifluoromethane sulfonate (68.3 mg, 0.23 mmol) in DMF (0.5 mL) was added dropwise over 4 mins. Stirring was continued with cooling for 0.25 hrs and then at ambient temperature for 0.66 hrs. The mixture was concentrated and the residue partitioned between EtOAc and $H_2O$. The ethyl acetate layer was washed ($H_2O$, brine), dried ($Na_2SO_4$) and concentrated to leave a brown oil. The oil was chromatographed on $SiO_2$ -(10 g) with $CH_2Cl_2$-acetone (200:25) to afford the title compound (25 mg, 25 %) as a viscous oil, with an estimated purity of 92% (HPLC). HPLC : rt 10.47 minutes (Waters $C_{18}$ radial pak cartridge) flow rate 2mL/min;. 45% pump A (90%$H_2$0-10%$CH_3CN$), 55% pump B (20%$H_2O$-80%$CH_3CN$). Detection at 254 nm. $^1H$ NMR (200 MHz, $CDCl_3$) δ 7.19 (s, 1H), 6.19 (m, 1H), 5.95 (m, 1H), 5.76 (m, 1H), 5.48 (s, 2H), 4.65 (m, 1H), 4.23 (m, 4H), 3.92 (m, 2H), 3.76 (m, 2H), 2.92 (m, 1H), 1.99 (s, 3H), 1.74 (m, 1H), 1.45 (m, 6H), 1.07 (m, 2H), 0.06 (s, 9H).

Method 2:

A solution of 2.5 M n-BuLi in hexane (2.7 mL, 6.75 mmol) was added dropwise to a cooled ($CO_2$/acetone bath) stirred solution of (±)-1-(4-β-hydroxycyclopent-2-ene-1-β-yl)-N3-(2-(trimethylsilyl)-ethoxymethyl)thymine (1.9 g, 5.61 mmol) in THF (15 mL) under argon. A solution of diethyl phosphonylmethyltrifluoromethane sulfonate (2.53 g, 8.41 mmol) in THF (2 mL) was added dropwise over 1 min. The reaction was stirred at approximately -70 °C for 0.25 hrs and then the solution was allowed to warm to 22 °C. The solution was cooled in an ice/salt bath mixture and stirred for a further 0.25 hrs. Saturated aqueous $NH_4Cl$ was added and the THF removed in vacuo. The aqueous mixture was extracted with EtOAc and the organic layer sequentially washed (dilute aqueous $NaHCO_3$, $H_2O$, brine) and dried ($Na_2SO_4$). Removal of the EtOAc left a brown oil (3 g) which was flash chromatographed on $SiO_2$ (120 g) with $CH_2Cl_2$-acetone (10:1) to afford an initial fraction (877 mgs) containing the title compound with an estimated purity of 88 % by HPLC and an additional fraction of 494 mgs containing the title compound with an estimated purity of 73 % by HPLC. The major contaminant was the starting alcohol.

**EXAMPLE G: (±)-1-(4-β-Phosphonylmethoxycyclopent-2-ene-1-β-yl)-N3-(2-(trimethylsilyl)-ethoxymethyl)thymine.**

Bromotrimethylsilane (1.3 mL, 9.67 mmol) was added dropwise over 1 min to a stirred solution of (±)-1-(4-β-diethylphosphonylmethoxycyclopent-2-ene-1-β-yl)-N3-(2-(trimethylsilyl)ethoxymethyl)thymine (315 mg, 0.645 mmol) in DMF (5 mL) at 22 °C and under argon. The solution was stirred at ambient temperature for 4 hrs and then was concentrated to dryness in vacuo. The residual brown oil was dissolved in DMF (5 mL) and the solution reconcentrated. A solution of the residue in $H_2O$ was applied to the Michel-Miller $C_{18}$ column. The column was eluted with $H_2O$ containing from 10 to 40% of $CH_3CN$. The appropriate eluates were combined and concentrated to leave the title compound (144 mg, 74%) as a viscous glass. $^1H$ NMR (200 MHz, $CDCl_3$) δ 7.20 (s, 1H), 6.23 (m, 1H), 5.93 (m, 1H), 5.66 (m, 1H), 5.41 (s, 2H), 4.59 (m, 1H), 3.85 (m, 2H),3.73 (m, 2H), 2.80 (m, 1H), 1.93 (s, 3H), 1.78 (m, 1H), 1.00 (m, 2H), 0.02 (s, 9H).

**EXAMPLE H: (±)-1-(4-β-Phosphonylmethoxycyclopent-2-ene-1-β-yl)thymine and (±)-1-(4-β-phosphonylmethoxycyclo pent-2-ene-1-β-yl)-N3-(hydroxymethyl)thymine.**

Bromotrimethylsilane (2.0 mL) was added dropwise to a stirred solution of (±)-1-(4-β-diethylphosphonomethoxycyclopent-2-ene-1-β-yl)-N3-(2-(trimethylsilyl)ethoxymethyl)thymine(494 mg, estimated purity of 73%) in DMF (5 mL) at 22 °C and under argon. The solution was stirred for 0.25 hrs and then was concentrated to dryness. The residue was dissolved in a mixture of EtOH (18 mL) and IN HCl (18 mL). The mixture was stirred at an oil bath temperature of 55 °C for 0.75 hrs and then was heated to reflux for 1 hr. The solution was concentrated and the residue dissolved in $H_2O$. The aqueous solution was washed with ether and concentrated. The concentrate was applied to the Michel-Miller $C_{18}$ column. The column was

eluted with $H_2O$ containing 3% $CH_3CN$. Two groups of eluates were combined and the $CH_3CN$ removed by concentration in vacuo. Removal of the $H_2O$ from each group by lyophilization provided (±)-1-(4,β-phosphonomethoxycyclopent-2-ene-1-β-yl)thymine as a colorless solid (25 mg). HPLC: rt 6.93 mins (Waters $C_{18}$ radial pak cartridge) flow rate 2mL/min 95% pump A (0.05 M of pH 4.3 ammonium phosphate buffer), 5% pump B(20%$H_2O$-80%$CH_3CN$). Detection at 254 nm.
$^1H$ NMR (360 MHz, DMSO-$D_6$) δ 11.25 (s, 1H), 7.20 (s,1H), 6.32 (m,1H), 5.94 (m, 1H),5.42 (m, 1H), 4.51 (m, 1H), 3.59 (m, 1H), 2.66 (m, 1H), 1.74 (s, 3H), 1.58 (m, 1H), and a mixture of the foregoing and (±)-1-(4-β-phosphonylmethoxycyclopent-2-ene-1-β-yl)-N3-(hydroxymethyl)thymine (25.8 mg). HPLC: rt 11.06 min containing 33% of the fully deprotected thymine derivative. The pH of an aqueous solution of this latter mixture was raised to 12, which removed the $N^3$ hydroxymethyl group.

**EXAMPLE I: (±)-1-(4-β-Phosphonomethoxycyclopent-2-ene-1-β-yl)thymine.**

Bromotrimethylsilane (3.6 mL) was added dropwise to a stirred solution of (±)-1-(4,β-diethylphosphonomethoxycyclopent-2-ene-1-β-yl)-N3-2-(trimethylsilyl)ethoxymethylthymine (860 mgs) in DMF (10 mL) at 22 °C and under argon. The solution was stirred for 2.5 hrs and concentrated. The residue was diluted with DMF and reconcentrated. A mixture of the residue in EtOH (25 mL) and 1 N HCl (25 mL) was heated to reflux for 1.5 hrs. The mixture was concentrated to dryness and the residue dissolved in $H_2O$ (5 mL). The pH of the solution was raised to 12.8 by the addition of 1 N NaOH. After several minutes the pH was lowered to 2.2 by the addition of 85% $H_3PO_4$. The solution was applied to the Michel-Miller $C_{18}$ column. The column was eluted with a mixture of $H_2O$-$CH_3CN$-HOAc (1000:40:3). The appropriate fractions were combined as determined by HPLC and concentrated to dryness. The residue was dissolved in $H_2O$ and the aqueous solution concentrated by lyophilization to afford the title compound as a colorless lyophilate. This lyophilate was combined with several others from previous small scale experiments and the combination recrystallized from $H_2O$ (1 mL) to afford colorless crystals of the title compound (56 mg). M. p. 116-118 °C.
Analysis: $C_{11}H_{15}N_2O_6P.H_2O$ (calc) C: 41.26, H: 5.35, N: 8.74; (found) C: 41.36, H: 5.03, N: 8.85.

**EXAMPLE J: (±)-1-(4-β-Hydroxycyclopent-2-ene-1-β-yl)-N4-(dimethylaminomethylidene) cytosine.**

N,N-Dimethylformamide dimethyl acetal (361 μL, 2.72 mmol) was added to a stirred mixture of (±)-1-(4-β-hydroxycyclopent-2-ene-1-β-yl)cytosine (500 mg, 2.59 mmol) in DMF (10 mL). The reaction was then heated for 1.5 hrs at an oil bath temperature of 70-80 °C, during which time solution occurred. The solution wae concentrated and the residual solid crystallized from $CH_2Cl_2$ with the addition of ether to provide peach crystals of the title compound (580 mg, 90%). M. p. 180-181 °C. Analysis: $C_{12}H_{16}N_4O_2$ (calc) C: 58.06, H: 6.50, N: 22.57; (found) C: 57.62, H: 6.47, N: 22.18. $^1H$ NMR (200 MHz, $d_6$ DMS0) δ 8.63 (s, 1H), 7.63 (d, 1H), 6.15 (m, 1H), 6.00 (d, 1H), 5.80 (m, 1H), 5.50 (m, 1H), 5.25(m, 1H, exchangeable), 4.69 (m, 1H), 3.19 (s, 3H), 3.03 (s, 3H), 2.96 (m, 1H), 1.35 (m, 1H).

**EXAMPLE K: (±)-1-(4-β-Diethylphosphonomethoxycyclopent-2-ene-1-β-yl)-N4-(dimethylaminomethylidene) cytosine.**

A solution of 2.5 M n-BuLi in hexane (0.8 mL, 2.01 mmol) was added dropwise to a stirred mixture of (±)-1-(4,β-hydroxycyclopent-2-ene-1-β-yl)-N4-(dimethylaminomethylidene) cytosine (500 mg, 2.01 mmol) in HMPT (3 mL) and THF (4 mL) which was cooled in a IPA/$CO_2$ bath maintained at -40 to -30 °C. Stirring was continued at -40 to -20 °C for 0.5 hrs. The solution was then cooled to -40 °C when a solution of diethylphosphonomethyltrifluoromethane sulfonate (755 mg, 2.52 mmol) in THF (2 mL) was added dropwise. The solution was allowed to warm to 22 °C during 1.5 hrs and was quenched by the addition of saturated aqueous $NH_4Cl$ (1 mL). The mixture was concentrated and the concentrate, which contained residual HMPT, was flash column chromatographed. The column was sequentially eluted with $CH_2Cl_2$ (200 mL) and then with $CH_2Cl_2$ containing 10% MEOH to afford the title compound (1.1 g) as a gum, with an estimated purity of 90.6% by HPLC. HPLC: rt 4.87 mins (Waters $C_{18}$ radial pak cartridge); flow rate 2mL/min. of 75% pump A (0.05 M of pH 5 ammonium phosphate buffer), 25% pump B (20% $H_2O$-80% $CH_3CN$).

**EXAMPLE L: (±)-1-(4-β-Phosphonomethoxycyclopent-2-ene-1,β-yl) cytosine.**

Bromotrimethylsilane (1.7 mL, 12.6 mmol) was added to a stirred solution of (±)-1-(4-β-diethylphosphonomethoxycyclopent-2-ene-1-β-yl)-N4-(dimethylaminomethylidene)cytosine (1 g, 2.51 mmol) in

DMF (5 mL) at 22 °C. The reaction was stirred for two hours and then concentrated to dryness. A solution of residue in $H_2O$ was applied to the Michel-Miller $C_{18}$ column. The column was eluted with 0.025 M of pH 5 ammonium phosphate buffer containing 2% $CH_3CN$. The appropriate eluates were combined and the $CH_3CN$ removed in vacuo. The aqueous solution was lyophilized and the residual solid was dissolved in water and the solution applied to the Michel-Miller $C_{18}$ column. The column was eluted with $H_2O$ to remove the inorganic salts. Elution with $H_2O$ containing 20% $CH_3CN$ provided the title compound (166mg, 23%) as a colorless lyophilate with an estimated purity of >99% by HPLC. HPLC: rt 5.49 mins (Waters $C_{18}$ radial pak cartridge): flow rate 2mL/min of 98% pump A (0.05 M of pH 5 ammonium phosphate buffer), 2% pump B (20% $H_2O$-80%$CH_3CN$). Detection at 254 nm. Crystallization from $H_2O$-EtOH provided the analytical sample. M. p. 238-240 °C (decomp). Analysis $C_{10}H_{14}N_3O_5P$ (calc) C: 41.83, H: 4.92, N: 14.64; (found) C: 41.19, H: 4.95, N: 14.52.
$^1H$ NMR (360 MHz, $d_6$ DMSO) $\delta$ 7.35 (d, 1H), 6.27 (d, 1H), 5.91 (d, 1H), 5.69 (d,1H), 5.50 (m, 1H), 4.52 (m, 1H), 3.56 (d, 2H), 2.67 (m, 1H), 1.44(m,1H).

**EXAMPLE M: (±)-1-(4-β-Phosphonomethoxycyclopentane-1-β-yl)cytosine.**

A mixture of (±)-1-(4-β-phosphonomethoxycyclopent-2-ene-1-β-yl)cytosine (20 mg) and 10% palladium on carbon (15 mg) in $H_2O$ (50 mL) was shaken with hydrogen at 50 p.s.i. for 0.25 hrs. The solution was filtered and the filtrate concentrated by lyophilization to afford the title compound as a colorless solid with an estimated purity of 94% by HPLC. HPLC: rt 12.22 mins (Waters $C_{18}$ radial pak cartridge); flow rate 1mL/min. of 98% pump A (0.05 M of pH 5 ammonium phosphate buffer), 2% pump B (80% $CH_3CN$-20%$H_2O$).
$^1H$ NMR (360 MHz, $D_2O$) $\delta$ 8.03 (d, 1H), 6.09 (d, 1H), 5.02 (m, 1H), 4.08 (m, 1H), 3.56 (m, 2H), 2.32 (m, 1H), 2.15 (m, 1H), 2.00 (m, 1H), 1.75 (s, 3H).

**EXAMPLE N: Trans-cyclopent-2-ene-1,4-diol.**

Trans-2-phenylthio-3-cyclopenten-1-ol(1.21 g, 6.3 mmol) was dissolved in $C_2Cl_2$ (8 mL) and cooled to 0 °C in an ice bath under nitrogen. meta-Chloroperbenzoic acid (1g, 5.9mmol) was added in small batches. An exothermic reaction occurred. The reaction mixture was left to stir at room temperature for 3 hrs, and then filtered and concentrated to give the sulfoxide trans-2-phenylsulfinyl-3-cyclopenten-1-ol as a pale yellow solid. The sulphoxide was used directly without further purific-ation. The sulphoxide was dissolved in methanol (5 mL) and trimethyl phosphite (1.17 g; 9.4 mmol) and the reaction mixture heated to reflux overnight under nitrogen. The reaction mixture was concentrated and then flash chromatographed to give the trans diol as a colorless oil. (240 mgs, 40% overall). $^1H$ NNR (360 MHz, $CDCl_3$) $\delta$ 2.1 (m, 2H $CH_2$); 5.0 (m, 2H, CHO); 6.05 (m, 2H, CH=CH). $^{13}C$ NMR(50.03 MHz, $CDCl_3$) 44.28 ($CH_2$); 76.99 (CHOH); 137.20 (CH=CH).

**EXAMPLE O: Trans-1-diethylphosphonomethoxy-2-(phenylthio)cyclopent-3-ene.**

Sodium hydride (60%) (460 mgs; 11.5 mmol) was added to a dry three neck 100 ml round bottom flask equipped with a nitrogen inlet and magnetic stirrer. The hydride was washed with hexane (2 x 25 mL) and ether (2 x 25 mL), and the solvent removed by syringe after each washing. After the last wash the last traces of ether were removed on the pump. THF (10 mL) was added to the sodium hydride and the suspension was cooled in a ice-salt bath under a nitrogen atmosphere. Trans-2-phenylthiocyclopent-3-en-1-ol (2g, 10.5 mmol) in THF (5mL) was then added dropwise over 5 mins; hydrogen evolution was observed. Once all the alcohol had been added, the reaction mixture was left to stir at 0 °C for 1 hr and at ambient temperature for 1 hr. The reaction mixture became very dark in color during this time.

The reaction mixture was then cooled in a ice-salt bath again and a solution of trifluoromethanesulphonyloxymethyldiethylphosphonate (3.43 g, 11.4 mmol) in THF (10 mL) was slowly added dropwise. After addition, the reaction mixture was stirred at 0 °C for 1 hr. A further 0.5 equivalents of triflate in THF were added and the reaction mixture then allowed to stir at ambient temperature for 0.5 hr.
Methanol (3 mL) was added followed by ethyl acetate (50 mL). The organic phase was washed with brine, dil. sodium bicarbonate, water, brine and then dried over $MgSO_4$. The dried solution was filtered and concentrated to leave a clear dark brown oil. The product was purified by flash column chromatography with 2% MeOH/ 98% $CH_2Cl_2$ as eluent. to leave a colorless oil (2.33 g; 66%). $^1H$ NMR (360 MHz, $CDCl_3$): $\delta$ 7.81-7.43 (m, 5H), 5.78 (b, 1H), 5.69 (q, 1H), 4.02-4.15 (m, 4H), 3.51-3.65 (m, 2H), 2.61-2.67 (m, 1H), 2.37 (d, 1H), 1.24-1.32 (m, 6H). $^{13}C$ NMR (50.03 MHz, $CDCl_3$) 131.97, 129.64, 128.93, 127.16, 126.78, 87.51 and

87.26, 64.65 and 61.29, 62.51 and 62.38, 58.07, 38.17 and 16.55 and 16.42.

**EXAMPLE P: Trans-1-diethylphosphonomethoxy-2-(phenylsulfinyl)-cyclopent-3-ene.**

The sulfide (2.1 g; 6.1 mmol) was dissolved in $CH_2Cl_2$ (30mL), under nitrogen, and cooled to 0 °C. A solution of mCPBA (1.2 g; 7 mmol) in $CH_2Cl_2$ (10 mL) was then added dropwise. The reaction mixture was then stirred at 0 °C for 1.5 hrs. The reaction mixture was concentrated and flash chromatographed using 5% MeOH/95% $CH_2Cl_2$ as eluent. The product was a white solid (2.1 g; 95%).

**EXAMPLE Q: Trans-1-diethylphosphonomethoxy-cyclopent-2-en-4-ol.**

The sulphoxide (1 g, 2.7 mmol) was dissolved in MeOH (3 mL) and trimethylphosphite (0.7 g, 5.6 mmol) added under a nitrogen atmosphere. The reaction mixture was heated to reflux for 4 hrs and then allowed to cool. A saturated solution of $NaHCO_3$ (3 mL) was added and the mixture stirred for ten minutes. The reaction mixture was then extracted with ethyl acetate (3 x 25 mL) and the organic washings dried ($MgSO_4$). The dried solution was filtered and concentrated to give a pale yellow oil. The desired product was obtained as a colorless oil (220 mg, 32 %) after flash chromatography of the yellow oil with 5% MeOH/95% $CH_2Cl_2$ eluent mixture. $^1H$ NMR (360 $MH_z$; $CDCl_3$): 6.05-6.15 (m, 1H), 5.85-5.95 (m, 1H), 4.93-4.96 (m, 1H), 4.77-4.80 (m, 1H), 4.05-4.14 (septet, 4H), 3.66-3.69 (dd, 2H), 2.10-2.17 (m, 1H), 1.89-1.96 (m, 1H) and 1.25-1.29 (b, 6H).
$^{13}C$ NMR (50.03 MHz; $CDCl_3$): 139.37, 133.11, 85.98 and 85.74, 75.71, 64.02 and 60.67, 62.45, 40.29 and 16.41.

**EXAMPLE R: cis-1-Diethylphosphonomethoxy-4-chloro-cyclopent-2-ene.**

Trans-1-Diethylphosphonomethoxycyclopent-2-en-4-ol (380 mg, 1.52 mmol) was dissolved in $CH_2Cl_2$ (5 mL) and the solution was cooled in an ice bath under nitrogen. Triethylamine (168 mg, 1.68 mmol) was added by syringe followed by dropwise addition of a solution of toluenesulphonyl chloride (320 mg, 1.68 mmol) in $CH_2Cl_2$ (1 mL). A catalytic amount of DMAP was also added and then the reaction mixture left to stir overnight. Concentrate to leave a pale yellow oil which was purified by flash column chromatography. The chloride was isolated as a colorless oil (88 mg, 21%). along with a substantial amount of starting material (220 mg, 58%). $^1H$ NMR (360 MHz, $CDCl_3$): 6.00-6.08 (m, 2H), 4.75-4.78 (m, 1H), 4.66-4.69 (m, 1H), 4.08-4.17 (m, 4H), 3.69-3.76 (m, 2H), 2.79-2.88 (quintet, 1H), 2.02-2.08 (dr, 1H, 1.29-1.39 (d, 6H).

**EXAMPLE S: cis-1-Diethylphosphonomethoxy-4-chlorocyclopent-2-ene.**

Trans-4-diethylphosphonomethoxycyclopent-2-en-4-ol (220 mg, 0.88 mmol) was dissolved in $CH_2Cl_2$ (2 mL) and cooled to 0 °C under nitrogen. Triethylamine (192 mg, 192 mmol) was added followed by methanesulphonyl chloride (220 mg, 192 mmol) and the reaction mixture left to stir for 0.5 hrs at 0 °C. The reaction mixture was allowed to warm to room temperature and MeOH was added. The aqueous solution was diluted with $CH_2Cl_2$ (10 mL) and washed with water; saturated $NaHCO_3$ and brine. The solution was dried ($MgSO_4$), filtered, concentrated and then purified by flash column chromatography with 5% MeOH/95% $CH_2Cl_2$ (144 mg, 62 %).

**EXAMPLE T: (±)-9-(4-β-Diethylphosphonomethoxycyclopent-2-en-1-β-yl)adenine.**

Adenine (235 mg, 1.74 mmol) was added to a suspension of sodium hydride (0.045g, 1.88 mmol) in DMF (15 mL) under a nitrogen atmosphere. The reaction mixture was warmed to 60 °C for 1 hr to form a thick, viscous white suspension. The reaction was then allowed to cool to room temperature and a suspension of sodium bromide (0.18 g, 1.74 mmol) and cis-1-diethylphosphonomethoxy-4-chlorocyclopent-2-ene (0.45 g; 1.68 mmol) added. The reactants were warmed to 60 °C under a nitrogen atmosphere for 2 hours.

The reaction mixture was allowed to cool and then concentrated in vacuo. The residue was dissolved in ethyl acetate (50 mL) and then washed with water and dilute hydrochloride acid (10%). The organic phase was then dried ($Na_2SO_4$). The solution was filtered and concentrated to leave a thick oil (300 mg). The product contained both the alpha and beta anomers from the condensation reaction in a 1:1 ratio determined by $^1H$ NMR. These two anomers were separated on an IBM instruments $^{18}C$ semi-preparative column using 35% methanol:65% ammonium acetate as eluent (adjusted to pH 7). The undesired product

(±)-9-(4-β-diethylphosphonomethoxycyclopent-2-ene-1-α-yl) adenine elutes first as a white solid. Analysis: $C_{15}H_{22}N_5O_4P.O.SH_2O$ (calc) C: 47.86, H: 5.90, N: 18.64; (found) C: 48.23, H 5.99, N, 18.64. $^1H$ NMR (200 MHz; $CDCl_3$): 8.4 (s, 1H); 7.65(S,1H); 7.25 (s, 1H), 6.4-6.5 (m, 1H), 6.2-6.25 (m, 1H), 5.8-5.9 (m, 1H), 5.0-5.1 (m, 1H), 4.15-4.25 (m, 4H), 3.75-3.85 (m, 2H), 2.55-2.7 (m, 1H), 2.25-2.4 (m, 1H) and 1.25-1.4 (t, 6H). $^{13}C$ NMR (90.53 MHz, $CDCl_3$): 155.49, 152.90, 149.75, 138.10, 136.54, 133.61, 119.88, 85.60 and 85.46, 63.92 and 62.00, 62.46 and 62.39, 38.94, 36.41 and 16.36 and 16.30.

(±)-a-(4-β-diethylphosphonomethoxycyclopent-2-ene-1-β-yl)adenine elutes off the column second. M.p. 85-87 °C. 1H NMR (200 MHz; $CDCl_3$): 8.37 (s, 1H); 6:39 (m, 1H); 6.15 (m, 1H); 5.65 (m, 1H); 4.2 (m, 4H); 3.85 (d, 2H); 2.95 (m, 1H); 2.00 (m, 1H) and 1.35 (m, 6H).

**EXAMPLE U: Trans-1-Diethylphosphonomethoxy-4-phenylsulfinylcyclopent-2-ene.**

Trans-1-diethylphosphonomethoxycyclopent-2-en-4-ol (0.4 g; 1.6 mmol) was dissolved in $CH_2Cl_2$ (6 mL) and cooled to 0 °C in an ice bath under a nitrogen atmosphere. Triethylamine (0.3 g; 2.9mmol) was then added followed by a solution of toluene sulfinyl chloride (0.4 g, 2.9 mmol) in $CH_2Cl_2$ (2 mL). The reaction mixture was stirred at 0°C for 1 hr. The ice bath was removed and the reaction mixutre concentrated in vacuo. The residual oil was purified by flash column chromotography with 2% methanol/98% dichloromethane as eluent. The product (0.54 g; 87%) was isolated as a colorless oil. Analysis: $C_{17}H_{25}O_6PS.O.5H_2O$ (calc): C: 51.37, H: 6.35; (found): C: 51.66, H: 6.54. Both the $^1H$ NMR and $^{13}C$ NMR show two diastereomers.

$^1H$ NMR (360 MHz; $CDCl_3$): 7.55-7.59 (m, 2x2H); 7.30-7.34 (m, 2x2H), 6.09-6.15 (m, 1x2H and 1x1H), 5.70-5.73 (m, 1x1H), 5.39-5.43 (1m, 2x1H), 4.80-4.82 (m,1x1H), 4.75-4.77 (m, 1x1H), 4.06-4.21 (m, 2x4H), 3.65-3.75 (m, 2x2H), 2.41 (s, 2x3H), 2.17-2.31 (m, 1x2H), 1.89-2.16 (m, 1x2H), 1.27-1.33 (m, 2x6H). $^{13}C$ NMR (50.03 MHz; $CDCl_3$): 136.5, 136.19, 135.77, 135.12, 129.67, 125.04, 85.45 and 85.19 with 85.36 and 85.11,-(2x1 C), 80.79 with 80.67 (2x1C), 64.47 and 61.15 with 64.41 and 61.07 (2x1C), 62.49 with 62.38 (2x1C), 38.68 with 38.12,(2x1C), 21.53, 16.50 with 16.39(2x1C).

**EXAMPLE V: (±)-9-(4-Diethylphosphonomethoxycyclopent-2-en-1-β-yl)adenine.**

Adenine (0.22 g, 1.6 mmol) was added to a suspension of sodium hydride (0.045g; 1.8 mmol) in DMF (5 mL) under nitrogen and the reaction warmed to 60 °C for 1 hr. The reaction was allowed to cool to room temperature and a solution of trans-1-diethylphosphonomethoxy-4-phenylsulfinylcyclopent-1-ene (0.6 g; 1.55 mmol) in DMF (2 mL) was then added by syringe. The reaction mixture was warmed to 70°C overnight. The reaction was allowed to cool, filtered to remove any solids and then concentrated in vacuo. The residue was purified by flash column chromatography using 5% methanol:95% dichloromethane as eluent. The product was isolated as a white solid.

M. p.: 80-91 °C

**EXAMPLE W: (±)-9-(4-β-Diethylphosphonomethoxycyclopent-2-en-1-β-yl)adenine.**

Trans-1-diethyl phosphonomethoxycyclopent-2-en-4-ol (0.02 g; 0.08 mmol) was dissolved in N-methylpyrrolidinone (1.5 mL) under a nitrogen atmosphere. To this triphenylphosphine (0.023 g; 0.09 mmol), diethyldiazodicarboxylate (0.016 g; 0.09 mmol) and adenine (0.011 g; 0.08mmol) were added in order and the reaction mixture left overnight. HPCL analysis shows product to be present. The reaction mixture was concentrated and then purified by flash column chromotography to give the product. This was identical to the product prepared by the method of Example V.

**EXAMPLE X: (±)-9-(4-β-Hydroxycyclopent-2-en-1-β-yl)adenine**

A solution of the 3,4-epoxycyclopentene (1.325 g, 15.8 mmol) in DMF (5 mL) was added dropwise to a suspension of adenine (1.74 g: 12.88 mmol) and tetrakis(triphenylphosphine)palladium (O) (0.74 g; 0.6mmol) in a DMF (25 mL) and THF (5 mL) mixture under a nitrogen atmosphere. The reaction mixture was stir red at ambient temperature for 1 hr and then warmed to 80-90 °C for a further 1 hr. The reaction mixture was poured onto water and then filtered. The filtrate was concentrated to leave a dark oil. The oil was purified by flash colum chromatography using a methanol/dichloromethane mixture as eluent (3% MeOH/ $CH_2Cl_2$ increasing to 5% MeoH/$CH_2Cl_2$). The product was isolated as a white solid. M. p. 164-167 °C. $^1H$ NMR: (360 MHz; $CDCl_3$) δ 8.26 (s, 1H), 7.83 (s, 1H), 6.33 (q, 1H), 5.80 (m, 1H), 5.26 (m, 1H), 4.84 (d, 1H), 2.97 (m, 1H), 2.23 (d, 1H).

$^{13}C$ NMR: (50.3 MHz, DMSO $d_6$) 156.02, 152.13, 143.62, 139.27, 139.24, 130.64, 119.03, 73.75, 57.12 and 41.08. M. S. M+H = 218.

**EXAMPLE Y: (±)-9-(4-β-Diethylphosphonomethyoxy-cyclopent-2-en-1-β-yl)adenine.**

(±)-9-(4-β-Hydroxycyclopent-2-en-1-β-yl)adenine (3.1 g, 14 mmol) in dry THF (30 mL) was added dropwise over 0.5 hrs to a stirred suspension of sodium hydride (97%) (0.376g, 15.7 mmol) in THF (10 mL) under nitrogen. Once the addition had been completed the reaction mixture was heated to reflux for 1 hr. The solution was allowed to cool to ambient temperature and then cooled to -70 °C with a dry ice/acetone bath. A solution of trifluoromethanesulfonyloxymethyldiethyl phosphonate (4.8 g, 16.0 mmol) in THF (30 mL) was then added dropwise over 0.25 hrs. The reaction mixture was stirred at -78 °C for 2 hrs and then allowed to warm to room temperature overnight. The solution was concentrated in vacuo to leave a thick oil. This oil was purified by flash column chromatography with a methanol/dichloromethane as eluent (5% $MeOH/CH_2Cl_2$ increasing to a 10% $MeOH/CH_2Cl_2$ mixture) to give the desired product as a beige colored solid (2.0 g, 40 %). M. p. 88-90 °C. Analysis: $C_{15}H_{22}NsO_4P$ (calc) C: 49.05, H: 6.04, N: 19.07; (found) C: 49.19, H: 5.96, N: 19.35. $^1H$ NMR: (350 $MH_z$, $CDCl_3$) δ 8.35 (s, 1H), 7.94 (s, 1H), 6.37 (m, 1H), 6.10 (m, 1H), 5.63 (m, 1H), 4.69 (m, 1H), 4.16 (quintet, 4H), 3.85 (m, 2H), 2.9 (m, 1H), 1.97 (d.t, 1H); 1.33 (m, 4H).
$^{13}C$ NMR (50.3 MHz, $CDCl_3$): 155.73, 152.89, 149.83, 139.58, 136.11, 134.02, 119.80, 84.82 (d, J = 12Hz), 64.58 (d, J = 167 Hz), 62.90 (d, J = 2.5), 62.77 (d, J = 2.5 Hz), 36.84, 33.76 and 16.76 (d, J = 5 Hz).

**EXAMPLE Z: (±)-9-(4-β-Monoethylphosphonomethoxycyclopent-2-en-1-β-yl)adenine.**

(±)-9-(4-8-Diethylphosphonomethoxycyclopent-2-en-1-β-yl)adenine (0.5g; 1.35 mmol) was dissolved in a solution of 1N sodium hydroxide (13 mL) and the reaction mixture stirred at room temperature for 2 hrs. The solution was acidified with 10% aqueous hydrochloric acid and then concentrated.
Residual salts were removed by reverse phase column chromatography (C18 adsorbent, elution with water) to give the product on a white solid (180 mg). M. p. = 192-205 °C. Analysis: $C_{13}H_{18}N_sO_4P$. $O.5H_20$ (calc) C: 44.83, H: 5.50, N: 20.11; (found): C: 45.31, H:5.37, N: 20.17.
$^1H$ NMR (300 Mhz: $d_6$ DMSO) 8.19 (s, 1H), 8.04 (s, 1H), 7.57 (brs, 1H), 6.30 (d, 1H), 6.18 (d, 1H), 5.50 (brs, 1H), 4.67 (brs, 1H), 3.95 (m, 2H), 3.74(m,2H), 2.87 (m, 1H), 1.90 (m, 1H), 1.22 (s, 3H). $^{13}C$ NMR (75.40 MHz, $d_6$ DMSO) 155.65, 151.92, 149.02, 138.78, 135.45, 133.33, 116.79, 83.73 (d, J = 13 Hz), 63.29 (d, J = 161 Hz), 60.68, 56.52, 37.76 and 16.35 (d, J = 5Hz).

**EXAMPLE AA: (±)-9-(4-β-Phosphonomethoxycyclopent-2-en-1-β-yl)adenine.**

Bromotrimethylsilane (2.1 g, 13.5mmol) was added to a solution of (±)-9-(4-β-diethylphosphonomethoxycyclopent-2-en-1-β-yl)adenine (0.5 g, 1.35 mmol) in dry DMF (5 mL) at room temperature under a nitrogen atmosphere. The reaction vessel was covered with foil. The reaction mixture was left to stir at room temperature for 4 hrs. The volatiles were then removed in vacuo to leave a thick oil. The residue was treated with water (2-3 mL) followed by acetone (2-3 mL). After standing for a few minutes a brown precipitate falls out. The precipate was collected and crystallized from water/acetone (80 mg). M. p. 218-220 °C (dec).
Analysis: $C_{11}H_{14}N_5O_4P.O.5H_2O$ (calc): C: 41.26, H: 4.42, N: 21.87; (found): C: 41.53, H: 4.49, N: 21.57.
$^1H$ NMR (360 $MH_z$: $d_6$ DMSO): 8.16 (s, 1H), 8.03 (s, 1H), 7.38 (s, 1H), 6.36 (d, 1H), 6.16 (d, 1H); 5.49 (brs, 1H), 4.66 (brs, 1H): 3.64 (d, 2H); 2.87 (m, 1H); 1.90 (d, 1H).
$^{13}C$ NMR (75.46 MHz; $d_6$ DMSO): 155.62, 151.93, 149.05, 138.80, 135.54, 133.13, 118.85, 83.62 (d, J = 5 Hz), 64.87 (d, J = 169 Hz), 56.46 and 37.61.

**EXAMPLE BB: (±)-9-(4-β-Diethylphosphonomethoxy-cyclopentan-1-β-yl)adenine.**

(±)-9-(4-β-Diethylphosphonomethoxy-2-cyclopentane-1-β-yl)adenine (0.4 g; 1.1 mmol) was dissolved in ethanol (10 mL) and $PtO_2$ catalyst (20 mg) added. The reaction was stirred under a hydrogen atmosphere for 18 hrs. The reaction mixture was filtered and purifed by flash column chromatography to give the product as a colorless oil (378 mg). Analysis: $C_{15}H^{24}N_SO_4P$.0.5 EtOH (calc): C: 48.96, H: 6.95, N: 17.84; (found): C: 48.93, H: 6.83, N: 17.48 $^1H$ NMR (200 $MH_z$; $CDCl_3$): 8.39 (s, 1H), 8.12 (d, 1H), 6.13 (brs, 1H), 5.18 (brs, 1H), 4.2-4.35 (m, 4H), 3.90 (m, 2H), 1.8-2.62 (set of m, 6H) and 1.37 (m, 6H). $^{13}C$ NMR (50.3 MHz; $CDCl_3$), 155.80, 152.38, 149.48, 138.99, 110.95, 82.88 (d, J = 13 Hz), 62.44 (d, J = 168 Hz), 62.35 d, J = 27 Hz), 61.96, 52.21, 39.18, 31.63, 30.68 and 16.45 (d, J = 5 hz).

**EXAMPLE CC: (±)-9-4-β-Phosphonomethoxycyclopentan-1-β-yl) adenine**

Bromotrimethylsilane (0.84 g; 5.5mmol) was added to a solution of (±)-9-4-β-diethylphosphonomethoxycyclopentan-1-β-yl)adenine (0.2 g; 0.55 mmol) in dry DMF (2 mL) at room temperature under a nitrogen atmosphere and the reaction mixture stirred for 12 hrs. The volatiles were removed in vacuo to leave a thick oil. Water was added and the oil went into solution. Acetone was then added and a precipitate fell out of solution. The precipitate was recrystallized from water/acetone. The product was a white solid. M. p. 255 °C (dec). Analysis: $C_{11}H_{16}N_SO_4P.0.5H_2O$ (calc): C: 42.08, H: 5.15, N: 22.36; (found): C: 42.36, H: 5.15, N: 22.20. $^1H$ NMR (200 MHz; $d_6$ DMSO) δ 8.25 (s, 1H), 8.19 (s,1H), 7.2(brs, 2H), 5.0 (m, 1H), 4.18 (m, 1H), 3.33 (d, 2H), 1.6-2.4 (m, 6H).

**EXAMPLE DD: (±)-6-0-Benzyl-9-(4-β-hydroxycyclopent-2-en-1-β-yl)guanine**

A solution of the 3.4-epoxycyclopentene (6.38 g; 77.7 mmol) in DMF (10 mL) was added dropwise over 0.66 hr to a prewarmed (67 °C) solution of 6-0-benzylguanine (12.5 g; 52.6 mmol) tetrakis-(triphenylphosphine)palladium (0) (3.0 g; 2.6 mmol) and triphenylphosphine (1.35 g; 5.51 mmol) in DMF (150 mL) . Once all the epoxide had been added the reaction was left at 75 °C overnight. The reaction mixture was then allowed to cool to room temperature. The reaction was filtered and then concentrated to a yellowish oil. This oil was purified by flash column chromatography using a methanol/dichloromethane mixture was eluent (1% $MeOH/CH_2Cl_2$ increasing to 10% $MeOH/CH_2Cl_2$) to give the product as a white solid (13.0 g, 77 %). M. p.: 110-115 °C.

Analysis; $C_{17}H_{17}N_5O_2.0.5H_2O$ (calc): C:61.44′ H: 5.46, N: 21.08, (found) 61.32, H: 5.43, N: 19.78. $^1H$ NMR-(360 MHz; $d_6$ DMSO + $D_20$): 7.76 (s, 1H), 7.29-7.46 (m, 5H), 6.13 (m, 1H), 5.89 (m, 1H), 5.47 (s, 1H), 5.25 (brs, 1H), 4.66 (brs, 1H), 2.69-2.85 (m, 1H) and 1.55-1.61(m, 1H). $^{13}C$ NMR (50.30 $MH_z$; $CDCl_3$) 161.15, 158.29, 152.50, 139.49, 139.38, 136.18, 129.89, 128.30, 128.16, 127.95, 116.77, 75.04, 68.01, 59.53 and 39.37.

**EXAMPLE EE: (±)-N2-monomethoxyltrityl-6-0-Benzyl-9-(4-β-hydroxycyclopent-2-en-1-β-yl)guanine.**

(±)-6-0-Benzyl-9-(4-β-hydroxycyclopent-2-en-1-β-yl)guanine (12.65 g; 39.12 mmol) was dissolved in DMF (200 mL) and anisylchlorodiphenylmethane (14.53 g; 46.94 mmol); triethylamine (9.8 g; 13.5 mL; 97.03 mmol) and dimethylaminopyridine (DMAP) (100 mg) were then added in turn. Once all the reactants had been added the reaction was stirred at room temperature overnight. The DMF was then removed in vacuo to leave a pale yellow oil which was purified by flash column chromatography with ethyl acetate/ hexane as eluent (10:1 EtOAc/ Hexane). The desired product was isolated as a colorless oil which became a foam on drying (21.5 g, 92 %). M.p. °C. Analysis $C_{17}H_{33}N_5O_3.1.0H_2O$: (calc) C: 72.43, H: 5.38, N: 11.41, (found) C: 72.43, H: 5.73, N: 12.06. $^1H$ NMR (360 MHz; $CDCl_3$) δ 7.99 (s. 1H), 7.36 (s, 1H), 7.14-7.29 (m, 17H), 6.73 (d, 2H), 6.23 (d, 1H), 5.77 (m, 1H), 5.10 (d, 1H), 4.76 (d, 1H), 4.70 (s, 2H), 3.79 (s. 3H), 2.79-2.80 (m, 1H), 2.06 (d, 1H).

**EXAMPLE FF: (±) - N2-monomethoxytrityl-6-O-benzyl-9-(4-β-diethylphosphonylmethoxycyclopent-2-en-1-β-yl)guanine.**

(±) - N2-monomethoxytrityl-6-O-benzyl-9-(4-β-hydroxycyclopent-2-en-1-β-yl)guanine (8.3 g; 13.93 mmol) was dissolved in DMF (90 mL) and stirred under argon. Sodium hydride (835 %; 52 mg; 27.86 mmol) was then added and the resulting slurry stirred at room temperature for 2.5 hr during which time the reaction mixture became dark brown. Diethyl tosyloxymethylphosphonate (6.7 g; 20.90 mmol) was introduced into the suspension via a syringe. THe reaction mixture was stirred at room temperature for 20 hr. during which time it became homogeneous. Ethanol (10 mL) was added and the reaction stirred for a further 0.3 hr. The solution was concentrated in vacuo and the residue flash chromatographed on silica with ethanol/ ethyl acetate (2 - 10 %) as eluent. The desired fractions were collected to afford the product (5.7 g; 57 %) as an off white foam after drying in vacuo. Analysis $C_{42}H_{44}N_5O_6P.0.5H_2O$: (calc) C: 66.84, H: 6.01, N: 9.28, (found) C: 67.09, H: 6.10, N: 9.01. $^1H$ NMR (300 MHz, $d_6$ DMSO) 7.56 (s, 1H), 7.45 - 7.13 (m, 17H), 6.81 (d, 2H), 6.26 (br s, 1H), 6.01 (br s, 1H), 5.03 (br m, 3H, H4′ plus $CH_2$), 4.56 (br s, 1H), 4.05 - 3.95 (m, 4H), 3.85 (d, J = 8 Hz, 2H), 3.68 (s, 3H), 2.60 (m, 1H), 1.70 (m, 1H), 1.18 (m, 6H).

**EXAMPLE GG: (±) 9-(4-β-Diethylphosphonylmethoxycyclopent-2-en-1-β-yl)guanine**

The product from example FF (3.65 g, 2.68 mmol) was dissolved in glacial acetic acid (90 mL), and the resulting yellow solution was heated on a steam bath with occasional swirling for 3 hr. The acetic acid was removed in vacuo and residue flash chromatographed on silica using methanol/dichloromethane (5 - 10 %) as eleuent. The desired product was obtained as a white solid (3.65 g, 68 %).
M. P. °C. Analysis $C_{15}H_{22}N_5O_5P$: (calc) C: 47.00, H: 5.79, N: 18.27, (found) C: 46.82, H: 6.00, N: 17.92. $^1$H NMR (360 MHz, $d_6$ DMSO) 7.31 (s, 1H), 6.27 (s, 2H), 6.16 (m, 1H), 5.96 (m, 1H), 5.05 (m, 1H), 4.43 (m, 1H), 3.85 (m, 4H), 3.72 (d, 2H), 2.59 (m, 1H), 1.64 (m, 1H), 1.05 (m, 6H). $^{13}$C NMR (50 MHz; $d_6$ DMSO)156.73, 153.46, 150.53, 135.16, 134.93, 133.86, 116.54, 83.83 (d, J = 13 Hz), 61.89 (d, J = 163 Hz), 61.73, 61.61, 56.00, 37.60, 16.20 (d, J = 6Hz).

**EXAMPLE HH: (±) 9-(4-β-Dihydroxyphosphonomethoxycyclopent-2-en-1-β-yl) guanine.**

(±) 9-(4-β-Diethylphosphonomethoxycyclopent-2-en-1-β-yl)guanine (1.15 g; 3.0 mmol) was placed in dry DMF (20 mL) to give a white slurry. Bromotrimethylsilane (3.9 mL) was added by syringe and the reaction mixture which immediately became homogeneous was stirred at ambient temperature for 20 hr. under argon. The yellow solution was concentrated in vacuo to remove the solvent residue (1 g) was dried under vacuum for 3 hr. The resultant foam was dissolved in $H_2O$ (5 mL) and stirred for 0.5 hr. Acetone was then added to the reaction mixture and a white precipitate came out of solution. The reaction was left sirring for 18 hr. The solution was filtered and thw white precipitate collected. The solid was then recrystallised from water to afford the product as a white crystalline solid (860 mg, 88 %) M. p. °C. Analysis $C_{11}H_{14}N_5O_5P.1.0H_2O$: (calc) C: 38.27, H: 4.68, N: 20.29, (found) C: 38.10, H: 4.76, N: 20.24.
$^1$H NMR (360 MHz, $d_6$ DMSO) 7.58 (s, 1H), 6.45 (s, 2H), 6.29 (m, 1H), 6.06 (m, 1H), 5.21 (br s, 1H), 4.60 (m, 1H), 3.62 (m, 2H), 2.77 (m, 1H), 1.78 (m, 1H). $^{13}$C NMR (75 MHz, $d_6$ DMSO) 156.57, 153.36, 150.45, 135.41, 133.26, 116.23, 83.57, 6418 (d, J = 161 Hz), 56.07, 37.64.

**Claims**

**Claims for the following Contracting States : AT, BE, CH, DE, FR, GB, IT, LI, LU, NL, SE**

1. A phosphonylmethoxy compound having Formula I or Formula II

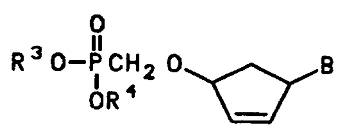

I

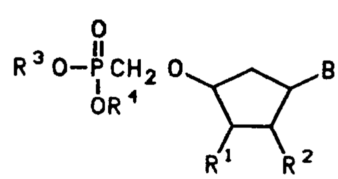

II

the phosphonylmethoxy group and B being in cis stereochemical relationship,
wherein

| | |
|---|---|
| $R^1$ and $R^2$ | are independently hydrogen, hydroxy, chlorine, bromine, or an organic substituent having 1 to 12 carbon atoms and selected from acyloxy, alkoxy, alkylthio, amino, alkylamino and dialkylamino, |
| $R^3$ and $R^4$ | are independently hydrogen, or organic substituents having 1 to 12 carbon atoms and selected from alkyl, alkenyl, aryl, and aralkyl, |
| B | is a heterocyclic group having at least one nitrogen heteroatom and up to three additional heteroatoms selected from nitrogen, oxygen and sulfur, said heterocyclic group being attached through a nitrogen heteroatom thereof, |

and the pharmaceutically acceptable acid addition, metal, and amine salts thereof.

2. The compound of Claim 1 wherein B is a heterocyclic group selected from purine, pyrimidine, azapurine, triazine, deazapurine, pyridine, and triazole, said heterocyclic group being substituted with from 1 to 3 substituents independently selected from oxo, hydroxy, amino, fluoro, chloro, bromo, iodo, alkyl having 1 to 3 carbon atoms, alkenyl having 2 to 3 carbon atoms, haloalkenyl having 2 to 3 carbon atoms, alkoxy having 1 to 3 carbon atoms, and alkylthiol having 1 to 3 carbon atoms.

3. The compound of Claim 2 wherein B is a heterocyclic group selected from, monosubstituted purine, or disubstituted purine.

4. The compound of Claim 2 wherein B is a heterocyclic group selected from monosubstituted pyrimidine, disubstituted pyrimidine, or trisubstituted pyrimidine.

5. The compound of Claim 1 having the 1β,4β-configuration.

6. The compounds of Claim 1: 1-[(1β,4β)-4-(dihydroxyphosphonyl)methoxycyclopent-2-en-1-yl]thymine,
1-[(1β,4β)-4-(dihydroxyphosphonyl)methoxycyclopent-2-en-1-yl]cytosine,
[(1β,4β)-4-(diethoxyphosphonyl)methoxycyclopent-2-en-1-yl]adenine,
9-[(1β,4β)-4-(ethoxyhydroxyphosphonyl)methoxycyclopent-2-en-1-yl]adenine,
9-[(1β,4β)-4-(dihydroxyphosphonyl)methoxycyclopent-2-en-1-yl]adenine,
9-[(1β,4β)-4-(diethoxyphosphonyl)methoxycyclopentan-1-yl]adenine,
9-[(1β,4β)-4-(dihydroxyphosphonyl)methoxycyclopentan-1-yl]adenine.

7. The compound of Claim 1 having the formula

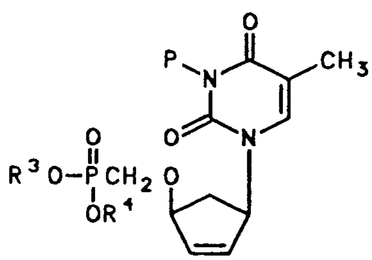

wherein $R^3$ and $R^4$ have the same definitions as given in Claim 1, and P is hydrogen or an amino protecting group.

8. The compound of Claim 1 having the formula

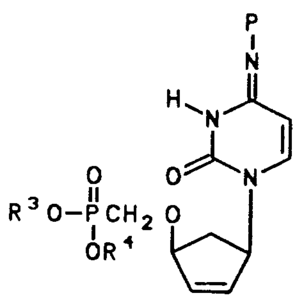

wherein $R^3$ and $R^4$ have the same definitions as in Claim 1 and P is hydrogen or an amino protecting group.

9. The compound of Claim 1 having the formula

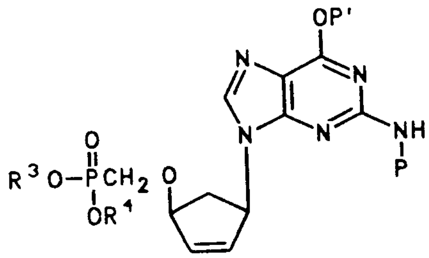

wherein $R^3$ and $R^4$ have the same definitions as in Claim 1, and P and P' are hydrogen or respectively amino and hydroxy protecting groups.

10. The process for preparing a compound of Claim 1, having Formula I which comprises etherifying a cyclopentenol derivative of Formula IV

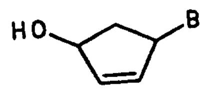

IV

with a phosphonylmethylating agent of Formula V

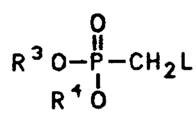

V

or aminating a phosphonylmethoxy cyclopentenol derivative of Formula VI

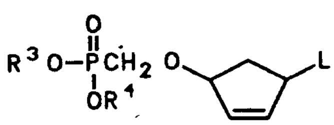

VI

with a nitrogen heterocycle of the formula BH, wherein $R^3$ and $R^4$ are organic substituents as defined in Claim 1, B is a heterocyclic group as defined in Claim 1, and the group L in Formulas V and VI is a leaving group.

11. The process for preparing a compound of Claim 1 having Formula II wherein $R^1$ and $R^2$ are each hydrogen, which comprises contacting a compound of Claim 1 having Formula I with hydrogen in the presence of a catalyst under reaction conditions appropriate for reducing a cyclopentene to a cyclopentane.

12. The process for producing a monoester phosphonic acid of Claim 1 having Formula I or Formula II wherein one of $R^3$ and $R^4$ is hydrogen and the other is an organic substituent, which comprises contacting a compound of Formula I or Formula II wherein $R^3$ and $R^4$ are each organic substituents with water in the presence of an alkali metal base at room temperature.

13. The process for preparing a phosphonic acid of Claim 1 having Formula I or Formula II wherein each of $R^3$ and $R^4$ is hydrogen which comprises contacting a compound having Formula I or Formula II wherein one or both of $R^3$ and $R^4$ is an organic substituent with trimethylsilyl bromide under appropriate reaction conditions.

14. A pharmaceutical composition comprising at least one compound according to anyone of claims 1 to 9, optionally in combination with pharmaceutically acceptable carriers and/or exhibients.

15. A process for preparing a pharmaceutical composition of claim 14 which comprises providing at least one compound of claims 1 to 9 in a form suitable for administration, optionally incorporated into a pharmaceutically acceptable carrier and/or excipient.

16. The use of a compound according to any one of claims 1 to 9 for preparing a pharmaceutical composition for treating viral diseases and diseases of microbial origin in animals, including man, or plants.

**Claims for the following Contracting State : ES**

1. A process for preparing a phosphonylmethoxycyclopentene having Formula I

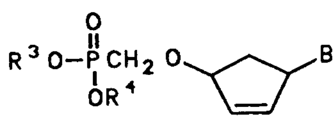

I

the phosphonylmethoxy group and B being in cis stereochemical relationship, wherein

- $R^3$ and $R^4$ are independently hydrogen, or organic substituents having 1 to 12 carbon atoms and selected from alkyl, alkenyl, aryl, and aralkyl,
- B is a heterocyclic group having at least one nitrogen heteroatom and up to three additional heteroatoms selected from nitrogen, oxygen and sulfur, said heterocyclic group being attached through a nitrogen heteroatom thereof,

and the pharmaceutically acceptable acid addition, metal, and amine salts thereof, which comprises etherifying a

cyclopentenol derivative of Formula IV

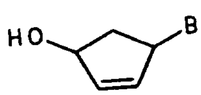

IV

with a phosphonylmethylating agent of Formula V

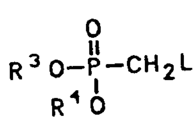

V

or aminating a phosphonylmethoxy cyclopentenol derivative of Formula VI

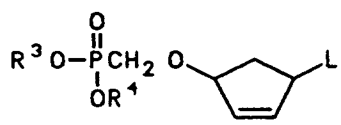

VI

with a nitrogen heterocycle of the formula BH, wherein $R^3$, $R^4$ and B are as defined above, and the group L in Formulas V and VI is a leaving group or
contacting a compound of formula I wherein $R^3$ and $R^4$ are organic substituents with water in the presence of an alkali metal base at room temperature to obtain a compound of formula I wherein one of $R^3$ and $R^4$ is hydrogen and the other is an organic substituent, or
contacting a compound having Formula I wherein one or both of $R^3$ and $R^4$ is an organic substituent with trimethylsilyl bromide under appropriate reaction conditions to obtain a compound of Formula I wherein $R^3$ and $R^4$ are hydrogen.

**2.** A process for preparing a phosphonylmethoxycyclopentene having Formula II

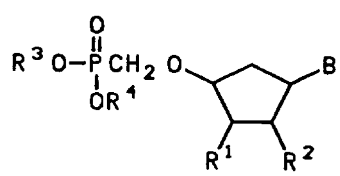

II

the phosphonylmethoxy group and B being in cis stereochemical relationship, wherein

| | |
|---|---|
| $R^1$ and $R^2$ | are independently hydrogen, hydroxy, chlorine, bromine, or an organic substituent having 1 to 12 carbon atoms and selected from acyloxy, alkoxy, alkylthio, amino, alkylamino and dialkylamino, |
| $R^3$ and $R^4$ | are independently hydrogen, or organic substituents having 1 to 12 carbon atoms and selected from alkyl, alkenyl, aryl, and aralkyl, |
| B | is a hetereocyclic group having at least one nitrogen heteroatom and up to three additional heteroatoms selected from nitrogen, oxygen and sulfur, said heterocyclic group being attached through a nitrogen heteroatom thereof, |

and the pharmaceutically acceptable acid addition, metal and amine salts thereof,
which comprises
contacting a compound of Formula I as defined in claim 1 with hydrogen in the presence of a catalyst under reaction conditions appropriate for reducing a cyclopentene to a cyclopentane to obtain a compound of Formula II wherein $R^1$ and $R^2$ are hydrogen, or
contacting a compound of Formula II wherein $R^3$ and $R^4$ are organic substituents with water in the presence of an alkali metal base at room temperature to obtain a compound of Formula II, wherein one of $R^3$ and $R^4$ is hydrogen and the other is an organic substituent, or
contacting a compound of Formula II wherein one or both of $R^3$ and $R^4$ is an organic substituent with trimethylsilyl bromide under appropriate reaction conditions to obtain a compound of formula II wherein $R^3$ and $R^4$ are hydrogen.

**3.** The process of Claim 1 or 2 wherein B is a heterocyclic group selected from purine, pyrimidine, azapurine, triazine, deazapurine, pyridine, and triazole, said heterocyclic group being substituted with from 1 to 3 substituents independently selected from oxo, hydroxy, amino, fluoro, chloro, bromo, iodo, alkyl having 1 to 3 carbon atoms, alkenyl having 2 to 3 carbon atoms, haloalkenyl having 2 to 3 carbon atoms, alkoxy having 1 to 3 carbon atoms, and alkylthiol having 1 to 3 carbon atoms.

**4.** The process of Claim 3 wherein B is a heterocyclic group selected from, monosubstituted purine, or disubstituted purine.

5. The process of Claim 3 wherein B is a heterocyclic group selected from monosubstituted pyrimidine, disubstituted pyrimidine, or trisubstituted pyrimidine.

6. The process of Claim 1 or 2 having the 1β,4β-configuration.

7. The process of Claim 1 or 2 for preparing the following compounds:
1-[(1β,4β)-4-(dihydroxyphosphonyl)methoxycyclopent-2-en-1-yl]thymine,
1-[(1β,4β)-4-(dihydroxyphosphonyl)methoxycyclopent-2-en-1-yl]cytosine,
[(1β,4β)-4-(diethoxyphosphonyl)methoxycyclopent-2-en-1-yl]adenine,
9-[(1β,4β)-4-(ethoxyhydroxyphosphonyl)methoxycyclopent-2-en-1-yl]adenine,
9-[(1β,4β)-4-(dihydroxyphosphonyl)methoxycyclopent-2-en-1-yl]adenine,
9-[(1β,4β)-4-(diethoxyphosphonyl)methoxycyclopentan-1-yl]adenine,
9-[(1β,4β)-4-(dihydroxyphosphonyl)methoxycyclopentan-1-yl]adenine.

8. The process of Claim 1 for preparing a compound having the formula

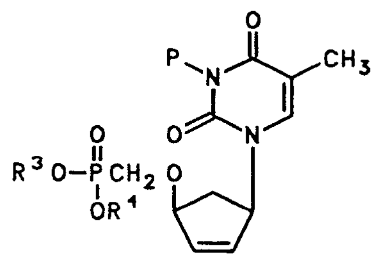

wherein $R^3$ and $R^4$ have the same definitions as given in Claim 1, and P is hydrogen or an amino protecting group.

9. The process of Claim 1 for preparing a compound having the formula

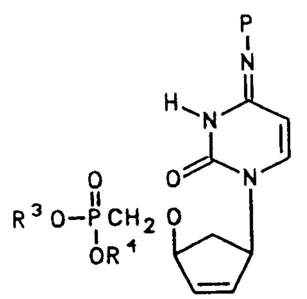

wherein $R^3$ and $R^4$ have the same definitions as in Claim 1 and P is hydrogen or an amino protecting group.

10. The process of Claim 1 for preparing a compound having the formula

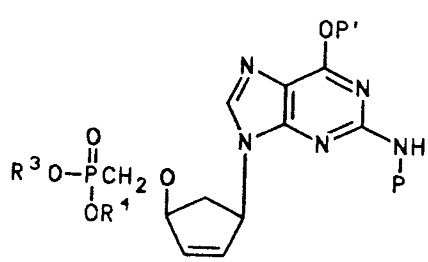

wherein $R^3$ and $R^4$ have the same definitions as in Claim 1, and P and P' are hydrogen or

respectively amino and hydroxy protecting groups.

**11.** A process for preparing a pharmaceutical composition which comprises providing at least one compound as defined in claims 1 to 10 in a form suitable for administration, optionally incorporated into a pharmaceutically acceptable carrier and/or excipient.

**Claims for the following Contracting State : GR**

**1.** A phosphonylmethoxy compound having Formula I or Formula II

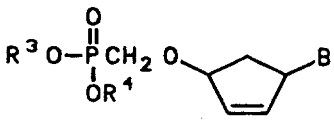

I

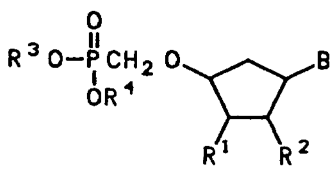

II

the phosphonylmethoxy group and B being in cis stereochemical relationship, wherein

- $R^1$ and $R^2$ are independently hydrogen, hydroxy, chlorine, bromine, or an organic substituent having 1 to 12 carbon atoms and selected from acyloxy, alkoxy, alkylthio, amino, alkylamino and dialkylamino,
- $R^3$ and $R^4$ are independently hydrogen, or organic substituents having 1 to 12 carbon atoms and selected from alkyl, alkenyl, aryl, and aralkyl,
- B is a heterocyclic group having at least one nitrogen heteroatom and up to three additional heteroatoms selected from nitrogen, oxygen and sulfur, said heterocyclic group being attached through a nitrogen heteroatom thereof,

and the pharmaceutically acceptable acid addition, metal, and amine salts thereof.

**2.** The compound of Claim 1 wherein B is a heterocyclic group selected from purine, pyrimidine, azapurine, triazine, deazapurine, pyridine, and triazole, said heterocyclic group being substituted with from 1 to 3 substituents independently selected from oxo, hydroxy, amino, fluoro, chloro, bromo, iodo, alkyl having 1 to 3 carbon atoms, alkenyl having 2 to 3 carbon atoms, haloalkenyl having 2 to 3 carbon atoms, alkoxy having 1 to 3 carbon atoms, and alkylthiol having 1 to 3 carbon atoms.

**3.** The compound of Claim 2 wherein B is a heterocyclic group selected from, monosubstituted purine, or disubstituted purine.

**4.** The compound of Claim 2 wherein B is a heterocyclic group selected from monosubstituted pyrimidine, disubstituted pyrimidine, or trisubstituted pyrimidine.

**5.** The compound of Claim 1 having the 1β,4β-configuration.

**6.** The compounds of Claim 1: 1-[(1β,4β)4-(dihydroxyphosphonyl)methoxycyclopent-2-en-1-yl]thymine,
1-[(1β,4β)-4-(dihydroxyphosphonyl)methoxycyclopent-2-en-1-yl]cytosine,
[(1β,4β)-4-(diethoxyphosphonyl)methoxycyclopent-2-en-1-yl]adenine,
9-[(1β,4β)-4-(ethoxyhydroxyphosphonyl)methoxycyclopent-2-en-1-yl]adenine,
9-[(1β,4β)-4-(dihydroxyphosphonyl)methoxycyclopent-2-en-1-yl]adenine,
9-[(1β,4β)-4-(diethoxyphosphonyl)methoxycyclopentan-1-yl]adenine,
9-[(1β,4β)-4-(dihydroxyphosphonyl)methoxycyclopentan-1-yl]adenine.

**7.** The compound of Claim 1 having the formula

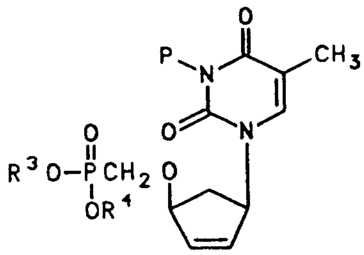

wherein $R^3$ and $R^4$ have the same definitions as given in Claim 1, and P is hydrogen or an amino protecting group.

**8.** The compound of Claim 1 having the formula

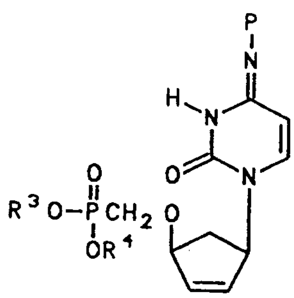

wherein $R^3$ and $R^4$ have the same definitions as in Claim 1 and P is hydrogen or an amino protecting group.

**9.** The compound of Claim 1 having the formula

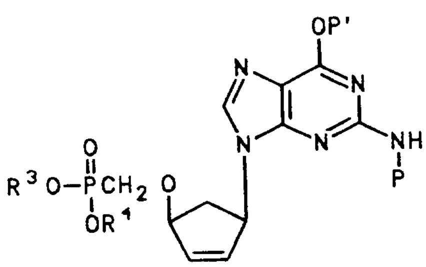

wherein $R^3$ and $R^4$ have the same definitions as in Claim 1, and P and P' are hydrogen or respectively amino and hydroxy protecting groups.

**10.** The process for preparing a compound of Claim 1, having Formula I which comprises etherifying a cyclopentenol derivative of Formula IV

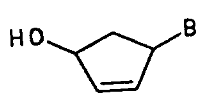

IV

with a phosphonylmethylating agent of Formula V

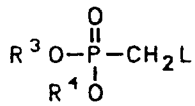

V

or aminating a phosphonylmethoxy cyclopentenol derivative of Formula VI

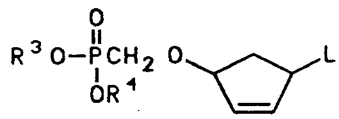

VI

with a nitrogen heterocycle of the formula BH, wherein $R^3$ and $R^4$ are organic substituents as defined in Claim 1, B is a heterocyclic group as defined in Claim 1, and the group L in Formulas V and VI is a leaving group.

**11.** The process for preparing a compound of Claim 1 having Formula II wherein $R^1$ and $R^2$ are each hydrogen, which comprises contacting a compound of Claim 1 having Formula I with hydrogen in the presence of a catalyst under reaction conditions appropriate for reducing a cyclopentene to a cyclopentane.

**12.** The process for producing a monoester phosphonic acid of Claim 1 having Formula I or Formula II wherein one of $R^3$ and $R^4$ is hydrogen and the other is an organic substituent, which comprises contacting a compound of Formula I or Formula II wherein $R^3$ and $R^4$ are each organic substituents with water in the presence of an alkali metal base at room temperature.

**13.** The process for preparing a phosphonic acid of Claim 1 having Formula I or Formula II wherein each of $R^3$ and $R^4$ is hydrogen which comprises contacting a compound having Formula I or Formula II wherein one or both of $R^3$ and $R^4$ is an organic substituent with trimethylsilyl bromide under appropriate reaction conditions.

**14.** A process for preparing a pharmaceutical composition which comprises providing at least one compound of claims 1 to 9 in a form suitable for administration, optionally incorporated into a pharmaceutically acceptable carrier and/or excipient.

**15.** The use of a compound according to any one of claims 1 to 9 for preparing a pharmaceutical composition for treating viral diseases and diseases of microbial origin in animals, including man, or plants.

**Patentansprüche**
**Patentansprüche für folgende Vertragsstaaten : AT, BE, CH, DE, FR, GB, IT, LI, LU, NL, SE**

**1.** Phosphonylmethoxyverbindung der Formel I oder der Formel II

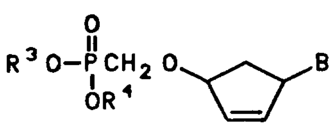

I

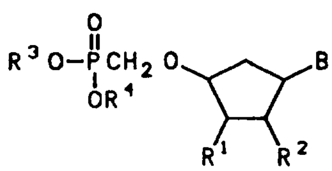

II

wobei die Phosphonylmethoxygruppe und B stereochemisch in cis-Stellung zueinander stehen, und worin

$R^1$ und $R^2$ unabhängig voneinander für Wasserstoff, Hydroxy, Chlor, Brom oder einen organischen Substituenten mit 1 bis 12 Kohlenstoffatomen, der ausgewählt ist unter Acyloxy, Alkoxy, Alkylthio, Amino, Alkylamino und Dialkylamino, stehen,

$R^3$ und $R^4$ unabhängig voneinander für Wasserstoff oder organische Substituenten mit 1 bis 12 Kohlenstoffatomen, die ausgewählt sind unter Alkyl, Alkenyl, Aryl und Aralkyl, stehen,

B eine heterocyclische Gruppe mit wenigstens einem Stickstoffheteroatom und bis zu drei weiteren Heteroatomen, ausgewählt unter Stickstoff, Sauerstoff und Schwefel, bedeutet, wobei die heterocyclische Gruppe über ein Stickstoffheteroatom gebunden ist,

und die pharmazeutisch akzeptablen Säureadditions-, Metall- und Aminsalze davon.

2. Verbindung nach Anspruch 1, wobei B für eine heterocyclische Gruppe steht, die ausgewählt ist unter Purin , Pyrimidin, Azapurin, Triazin, Deazapurin, Pyridin und Triazol, wobei die heterocyclische Gruppe mit 1 bis 3 Substituenten substituiert ist, die unabhängig voneinander ausgewählt sind unter Oxo, Hydroxy, Amino, Fluor, Chlor, Brom, Iod, Alkyl mit 1 bis 3 Kohlenstoffatomen, Alkenyl mit 2 bis 3 Kohlenstoffatomen, Halogenalkenyl mit 2 bis 3 Kohlenstoffatomen, Alkoxy mit 1 bis 3 Kohlenstoffatomen und Alkylthiol mit 1 bis 3 Kohlenstoffatomen.

3. Verbindung nach Anspruch 2, wobei B für eine heterocyclische Gruppe steht, die ausgewählt ist unter monosubstituiertem Purin oder disubstituiertem Purin.

4. Verbindung nach Anspruch 2, wobei B für eine heterocyclische Gruppe steht, die ausgewählt ist unter monosubstituiertem Pyrimidin, disubstituiertem Pyrimidin oder trisubstituiertem Pyrimidin.

5. Verbindung nach Anspruch 1 mit 1β,4β-Konfiguration.

6. Verbindungen nach Anspruch 1:
1-[(1β,4β)-4-Dihydroxyphosphonyl)methoxycyclopent-2-en-1-yl]thymin,
1-[(1β,4β)-4-Dihydroxyphosphonyl)methorycyclopent-2-en-1-yl]cytosin,
1-[(1β,4β)-4-Diethoxyphosphonyl)methoxycyclopent-2-en-1-yl]adenin,
9-[(1β,4β)-4-Ethoxyhydroxyphosphonyl)methoxycyclopent-2-en-1-yl]adenin,
9-[(1β,4β)-4-Dihydroxyphosphonyl)methoxycyclopent-2-en-1-yl]adenin,
9-[(1β,4β)-4-Diethoxyphosphonyl)methoxycyclopentan-1-yl]adenin,
9-[(1β,4β)-4-Dihydroxyphosphonyl)methoxycyclopentan-1-yl]adenin.

7. Verbindung nach Anspruch 1 der Formel

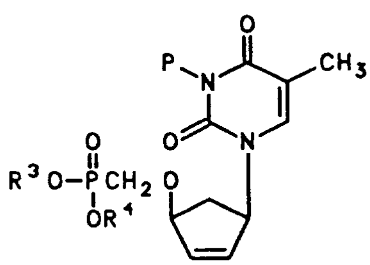

worin $R^3$ und $R^4$ die in Anspruch 1 angegebenen Bedeutungen besitzen und P für Wasserstoff oder eine Aminoschutzgruppe steht.

**8.** Verbindung nach Anspruch 1 der Formel

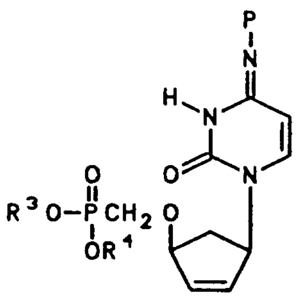

worin $R^3$ und $R^4$ die in Anspruch 1 angegebenen Bedeutungen besitzen und P für Wasserstoff oder eine Aminoschutzgruppe steht.

**9.** Verbindung nach Anspruch 1 der Formel

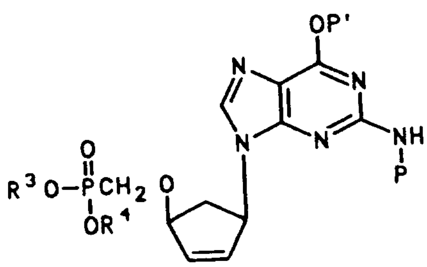

worin $R^3$ und $R^4$ die in Anspruch 1 angegebenen Bedeutungen besitzen und P und P' für Wasserstoff oder Amino- bzw. Hydroxyschutzgruppen stehen.

**10.** Verfahren zur Herstellung einer Verbindung nach Anspruch 1 der Formel I, wobei man ein Cyclopentenolderivat der Formel IV

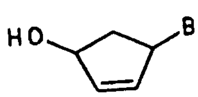

IV

mit einem Phosphonylmethylierungs-Mittel der Formel V

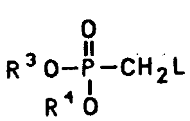

V

verethert oder ein Phosponylmethoxycyclopentenol-Derivat der Formel VI

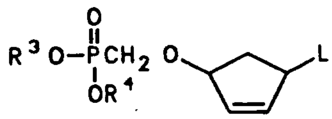

VI

mit einem Stickstoffheterozyklus der Formel BH aminiert, wobei $R^3$ und $R^4$ wie in Anspruch 1 definierte organische Substituenten bedeuten, B für eine wie in Anspruch 1 definierte heterocyclische Gruppe steht und die Gruppe L in den Formeln V und VI eine Abgangsgruppe ist.

**11.** Verfahren zur Herstellung einer Verbindung nach Anspruch 1 der Formel II, worin $R^1$ und $R^2$ jeweils für Wasserstoff stehen, wobei man eine Verbindung nach Anspruch 1 der Formel I mit Wasserstoff in Anwesenheit eines Katalysators unter Reaktionsbedingungen in Kontakt bringt, die zur Reduktion eines Cyclopentens zu einem Cyclopentan geeignet sind.

**12.** Verfahren zur Herstellung eines Phosphonsäuremonoesters nach Anspruch 1 der Formel I oder der Formel II, worin einer der Reste $R^3$ und $R^4$ für Wasserstoff steht und der andere einen organischen Substituenten bedeutet, wobei man eine Verbindung der Formel I oder der Formel II, worin $R^3$ und $R^4$ jeweils organische Substituenten bedeuten, mit Wasser in Anwesenheit einer Alkalimetallbase bei Raumtemperatur in Kontakt bringt.

**13.** Verfahren zur Herstellung einer Phosphonsäure nach Anspruch 1 der Formel I oder der Formel II, worin jeder der Reste $R^3$ und $R^4$ für Wasserstoff steht, wobei man eine Verbindung der Formel I oder der Formel II, worin einer oder beide der Reste $R^3$ und $R^4$ für einen organischen Substituenten stehen, mit Trimethylsilylbromid unter geeigneten Reaktionsbedingungen in Kontakt bringt.

**14.** Pharmazeutisches Mittel, umfassend wenigstens eine Verbindung nach einem der Ansprüche 1 bis 9, gegebenenfalls in Kombination mit pharmazeutisch akzeptablen Trägern und/oder Exzipienten.

**15.** Verfahren zur Herstellung eines pharmazeutischen Mittels nach Anspruch 14, wobei man wenigstens eine Verbindung der Ansprüche 1 bis 9 in einer zur Verabreichung geeigneten Form, gegebenenfalls aufgenommen in einem pharmazeutisch akzeptablen Träger und/oder Exzipienten, bereitstellt.

**16.** Verwendung einer Verbindung nach einem der Ansprüche 1 bis 9 zur Herstellung eines pharmazeutischen Mittels zur Behandlung viraler Erkrankungen und Erkrankungen mikrobiellen Ursprungs bei Tieren, einschließlich des Menschen, oder Pflanzen.

**Patentansprüche für folgenden Vertragsstaat : ES**

**1.** Verfahren zur Herstellung einer Phosphonylmethoxyverbindung der Formel I

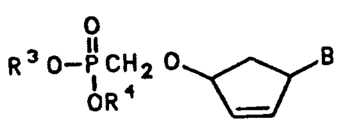

I

wobei die Phosphonylmethoxygruppe und B stereochemisch in cis-Stellung zueinander stehen, und worin

| | |
|---|---|
| $R^3$ und $R^4$ | unabhängig voneinander für Wasserstoff oder organische Substituenten mit 1 bis 12 Kohlenstoffatomen, die ausgewählt sind unter Alkyl, Alkenyl, Aryl und Aralkyl, stehen, |
| B | eine heterocyclische Gruppe mit wenigstens einem Stickstoffheteroatom und bis zu |

drei weiteren Heteroatomen, ausgewählt unter Stickstoff, Sauerstoff und Schwefel, bedeutet, wobei die heterocyclische Gruppe über ein Stickstoffheteroatom gebunden ist,
und der pharmazeutisch akzeptablen Säureadditions-, Metall- und Aminsalze davon, wobei man ein Cyclopentenolderivat der Formel IV

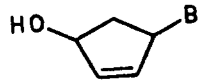

IV

mit einem Phosponylmethylierungs-Mittel der Formel V

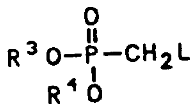

V

verethert oder
ein Phosphonylmethoxycyclopentenol-Derivat der Formel VI

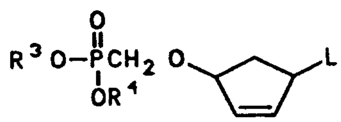

VI

mit einem Stickstoffheterozyklus der Formel BH aminiert, wobei $R^3$, $R^4$ und B wie oben definiert sind und die Gruppe L in den Formeln V und VI eine Abgangsgruppe ist, oder
eine Verbindung der Formel I, worin $R^3$ und $R^4$ organische Substituenten bedeuten, mit Wasser in Anwesenheit einer Alkalimetallbase bei Raumtemperatur in Kontakt bringt, wobei man eine Verbindung der Formel I erhält, worin einer der Reste
$R^3$ und $R^4$ für Wasserstoff steht und der andere ein organischer Phosphonestersubstituent ist, oder
eine Verbindung der Formel I, worin einer oder beide der Reste $R^3$ und $R^4$ für einen organischen Substituenten stehen, mit Trimethylsilylbromid unter geeigneten Reaktionsbedingungen in Kontakt bringt, wobei man eine Verbindung der Formel I erhält, worin $R^3$ und $R^4$ für Wasserstoff stehen.

**2.** Verfahren zur Herstellung eines Phosphonylmethoxycyclopentens der Formel II

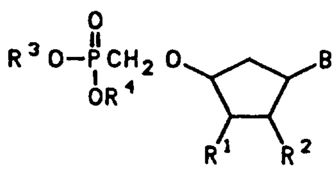

II

wobei die Phosphonylmethoxygruppe und B stereochemisch in cis-Stellung zueinander stehen, und worin
$R^1$ und $R^2$ unabhängig voneinander für Wasserstoff, Hydroxy, Chlor, Brom oder einen organischen Substituenten mit 1 bis 12 Kohlenstoffatomen, der ausgewählt ist unter Acyloxy, Alkoxy, Alkylthio, Amino, Alkylamino und Dialkylamino, stehen,

$R^3$ und $R^4$ unabhängig voneinander für Wasserstoff oder organische Substituenten mit 1 bis 12 Kohlenstoffatomen, die ausgewählt sind unter Alkyl, Alkenyl, Aryl und Aralkyl, stehen,

B eine heterocyclische Gruppe mit wenigstens einem Stickstoffheteroatom und bis zu drei weiteren Heteroatomen, ausgewählt unter Stickstoff, Sauerstoff und Schwefel, bedeutet, wobei die heterocyclische Gruppe über ein Stickstoffheteroatom gebunden ist,

und der pharmazeutisch akzeptablen Säureadditions-, Metall- und Aminsalze davon,

wobei man eine wie in Anspruch 1 definierte Verbindung der Formel I mit Wasserstoff in Anwesenheit eines Katalysators unter Reaktionsbedingungen in Kontakt bringt, die zur Reduktion eines Cyclopentens zu einem Cyclopentan geeignet sind, wobei man eine Verbindung der Formel II erhält, worin $R^1$ und $R^2$ für Wasserstoff stehen oder

eine Verbindung der Formel II, worin $R^3$ und $R^4$ organische Substituenten sind, mit Wasser in Anwesenheit einer Alkalimetallbase in Kontakt bringt, wobei man eine Verbindung der Formel II erhält, worin einer der Reste $R^3$ und $R^4$ für Wasserstoff steht und der andere einen organischen Substituenten bedeutet, oder

eine Verbindung der Formel II, worin einer oder beide der Reste $R^3$ und $R^4$ für einen organischen Substituenten stehen, mit Trimethylsilylbromid unter geeigneten Reaktionsbedingungen in Kontakt bringt, wobei man eine Verbindung der Formel II erhält, worin $R^3$ und $R^4$ für Wasserstoff stehen.

**3.** Verfahren nach Anspruch 1 oder 2, wobei B für eine heterocyclische Gruppe steht, die ausgewählt ist unter Purin , Pyrimidin, Azapurin, Triazin, Deazapurin, Pyridin und Triazol, wobei die heterocyclische Gruppe mit 1 bis 3 Substituenten subtituiert ist, die unabhängig voneinander ausgewählt sind unter Oxo, Hydroxy, Amino, Fluor, Chlor, Brom, Iod, Alkyl mit 1 bis 3 Kohlenstoffatomen, Alkenyl mit 2 bis 3 Kohlenstoffatomen, Halogenalkenyl mit 2 bis 3 Kohlenstoffatomen, Alkoxy mit 1 bis 3 Kohlenstoffatomen und Alkylthiol mit 1 bis 3 Kohlenstoffatomen.

**4.** Verfahren nach Anspruch 3, wobei B für eine heterocyclische Gruppe steht, die ausgewählt ist unter monosubstituiertem Purin oder disubstituiertem Purin.

**5.** Verfahren nach Anspruch 3, wobei B für eine heterocyclische Gruppe steht, die ausgewählt ist unter monosubstituiertem Pyrimidin, disubstituiertem Pyrimidin oder trisubstituiertem Pyrimidin.

**6.** Verfahren nach Anspruch 1 oder 2 mit 1β,4β-Konfiguration.

**7.** Verfahren nach Anspruch 1 oder 2 zur Herstelung folgender Verbindungen:
1-[(1β,4β)-4-Dihydroxyphosphonyl)methoxycyclopent-2-en-1-yl]thymin,
1-[(1β,4β)-4-Dihydroxyphosphonyl)methoxycyclopent-2-en-1-yl]cytosin,
1-[(1β,4β)-4-Diethoxyphosphonyl)methoxycyclopent-2-en-1-yl]adenin,
9-[(1β,4β)-4-Ethoxyhydroxyphosphonyl)methoxycyclopent-2-en-1-yl]adenin,
9-[(1β,4β)-4-Dihydroxyphosphonyl)methorycyclopent-2-en-1-yl]adenin,
9-[(1β,4β)-4-Diethoxyphosphonyl)methoxycyclopentan-1-yl]adenin,
9-[(1β,4β)-4-Dihydroxyphosphonyl)methoxycyclopentan-1-yl]adenin.

**8.** Verfahren nach Anspruch 1 zur Herstellung einer Verbindung der Formel

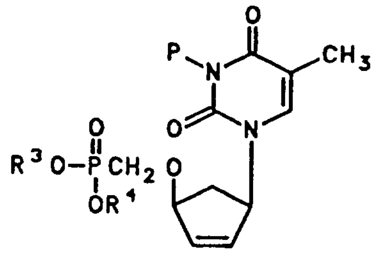

worin $R^3$ und $R^4$ die in Anspruch 1 angegebenen Bedeutungen besitzen und P für Wasserstoff oder

eine Aminoschutzgruppe steht.

**9.** Verfahren nach Anspruch 1 zur Herstellung einer Verbindung der Formel

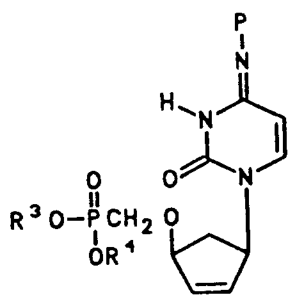

worin $R^3$ und $R^4$ die in Anspruch 1 angegebenen Bedeutungen besitzen und P für Wasserstoff oder eine Aminoschutzgruppe steht.

**10.** Verfahren nach Anspruch 1 zur Herstellung einer Verbindung der Formel

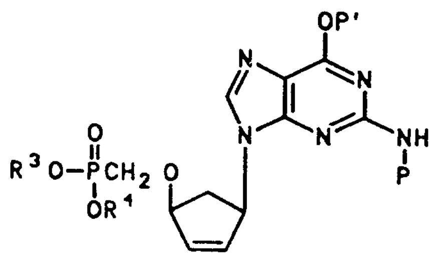

worin $R^3$ und $R^4$ die in Anspruch 1 angegebenen Bedeutungen besitzen und P und P' für Wasserstoff oder Amino- bzw. Hydroxyschutzgruppen stehen.

**11.** Verfahren zur Herstellung eines pharmazeutischen Mittels, wobei man wenigstens eine wie in den Ansprüchen 1 bis 10 definierte Verbindung in einer zur Verabreichung geeigneten Form, gegebenenfalls aufgenommen in einem pharmazeutisch akzeptablen Träger und/oder Exzipienten, bereitstellt.

**Patentansprüche für folgenden Vertragsstaat : GR**

**1.** Phosphonylmethoxyverbindung der Formel I oder der Formel II

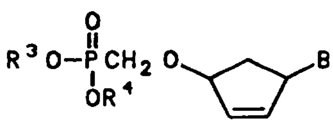

I

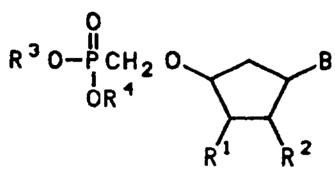

II

wobei die Phosphonylmethoxygruppe und B stereochemisch in cis-Stellung zueinander stehen, und worin

$R^1$ und $R^2$ unabhängig voneinander für Wasserstoff, Hydroxy, Chlor, Brom oder einen organischen Substituenten mit 1 bis 12 Kohlenstoffatomen, der ausgewählt ist unter Acyloxy, Alkoxy, Alkylthio, Amino, Alkylamino und Dialkylamino, stehen,

$R^3$ und $R^4$ unabhängig voneinander für Wasserstoff oder organische Substituenten mit 1 bis 12

Kohlenstoffatomen, die ausgewählt sind unter Alkyl, Alkenyl, Aryl und Aralkyl, stehen,

B eine heterocyclische Gruppe mit wenigstens einem Stickstoffheteroatom und bis zu drei weiteren Heteroatomen, ausgewählt unter Stickstoff, Sauerstoff und Schwefel, bedeutet, wobei die heterocyclische Gruppe über ein Stickstoffheteroatom gebunden ist,

und die pharmazeutisch akzeptablen Säureadditions-, Metall- und Aminsalze davon.

**2.** Verbindung nach Anspruch 1, wobei B für eine heterocyclische Gruppe steht, die ausgewählt ist unter Purin , Pyrimidin, Azapurin, Triazin, Deazapurin, Pyridin und Triazol, wobei die heterocyclische Gruppe mit 1 bis 3 Substituenten substituiert ist, die unabhängig voneinander ausgewählt sind unter Oxo, Hydroxy, Amino, Fluor, Chlor, Brom, Iod, Alkyl mit 1 bis 3 Kohlenstoffatomen, Alkenyl mit 2 bis 3 Kohlenstoffatomen, Halogenalkenyl mit 2 bis 3 Kohlenstoffatomen, Alkoxy mit 1 bis 3 Kohlenstoffatomen und Alkylthiol mit 1 bis 3 Kohlenstoffatomen.

**3.** Verbindung nach Anspruch 2, wobei B für eine heterocyclische Gruppe steht, die ausgewählt ist unter monosubstituiertem Purin oder disubstituiertem Purin.

**4.** Verbindung nach Anspruch 2, wobei B für eine heterocyclische Gruppe steht, die ausgewählt ist unter monosubstituiertem Pyrimidin, disubstituiertem Pyrimidin oder trisubstituiertem Pyrimidin.

**5.** Verbindung nach Anspruch 1 mit 1β,4β-Konfiguration.

**6.** Verbindungen nach Anspruch 1:
1-[(1β,4β)-4-Dihydroxyphosphonyl)methoxycyclopent-2-en-1-yl]thymin,
1-[(1β,4β)-4-Dihydroxyphosphonyl)methoxycyclopent-2-en-1-yl]cytosin,
1-[(1β,4β)-4-Diethoxyphosphonyl)methoxycyclopent-2-en-1-yl]adenin,
9-[(1β,4β)-4-Ethoxyhydroxyphosphonyl)methoxycyclopent-2-en-1-yl]adenin,
9-[(1β,4β)-4-Dihydroxyphosphonyl)methoxycyclopent-2-en-1-yl]adenin,
9-[(1β,4β)-4-Diethoxyphosphonyl)methoxycyclopentan-1-yl]adenin,
9-[(1β,4β)-4-Dihydroxyphosphonyl)methoxycyclopentan-1-yl]adenin.

**7.** Verbindung nach Anspruch 1 der Formel

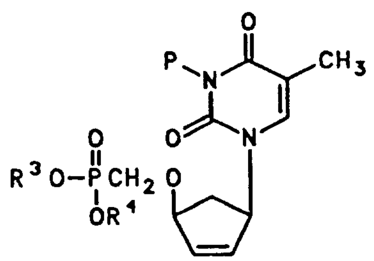

worin $R^3$ und $R^4$ die in Anspruch 1 angegebenen Bedeutungen besitzen und P für Wasserstoff oder eine Aminoschutzgruppe steht.

**8.** Verbindung nach Anspruch 1 der Formel

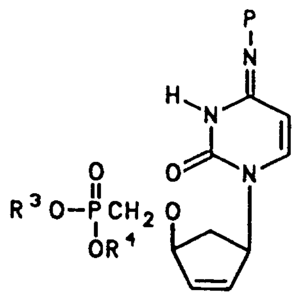

worin $R^3$ und $R^4$ die in Anspruch 1 angegebenen Bedeutungen besitzen und P für Wasserstoff oder eine Aminoschutzgruppe steht.

**9.** Verbindung nach Anspruch 1 der Formel

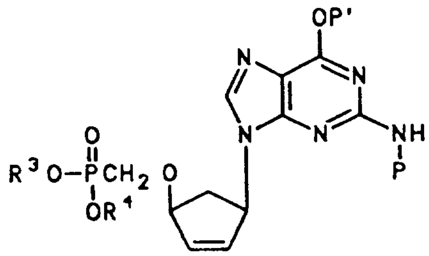

worin $R^3$ und $R^4$ die in Anspruch 1 angegebenen Bedeutungen besitzen und P und P' für Wasserstoff oder Amino- bzw. Hydroxyschutzgruppen stehen.

**10.** Verfahren zur Herstellung einer Verbindung nach Anspruch 1 der Formel I, wobei man ein Cyclopentenolderivat der Formel IV

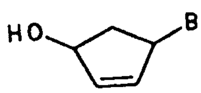

IV

mit einem Phosphonylmethylierungs-Mittel der Formel V

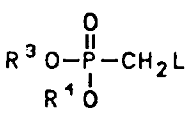

V

verethert oder ein Phosponylmethoxycyclopentenol-Derivat der Formel VI

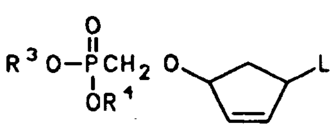

VI

mit einem Stickstoffheterozyklus der Formel BH aminiert, wobei $R^3$ und $R^4$ wie in Anspruch 1 definierte organische Substituenten bedeuten, B für eine wie in Anspruch 1 definierte heterocyclische Gruppe steht und die Gruppe L in den Formeln V und VI eine Abgangsgruppe ist.

**11.** Verfahren zur Herstellung einer Verbindung nach Anspruch 1 der Formel II, worin $R^1$ und $R^2$ jeweils für Wasserstoff stehen, wobei man eine Verbindung nach Anspruch 1 der Formel I mit Wasserstoff in Anwesenheit eines Katalysators unter Reaktionsbedingungen in Kontakt bringt, die zur Reduktion eines Cyclopentens zu einem Cyclopentan geeignet sind.

12. Verfahren zur Herstellung eines Phosphonsäuremonoesters nach Anspruch 1 der Formel I oder der Formel II, worin einer der Reste $R^3$ und $R^4$ für Wasserstoff steht und der andere einen organischen Substituenten bedeutet, wobei man eine Verbindung der Formel I oder der Formel II, worin $R^3$ und $R^4$ jeweils organische Substituenten bedeuten, mit Wasser in Anwesenheit einer Alkalimetallbase bei Raumtemperatur in Kontakt bringt.

13. Verfahren zur Herstellung einer Phosphonsäure nach Anspruch 1 der Formel I oder der Formel II, worin jeder der Reste $R^3$ und $R^4$ für Wasserstoff steht, wobei man eine Verbindung der Formel I oder der Formel II, worin einer oder beide der Reste $R^3$ und $R^4$ für einen organischen Substituenten stehen, mit Trimethylsilylbromid unter geeigneten Reaktionsbedingungen in Kontakt bringt.

14. Verfahren zur Herstellung eines pharmazeutischen Mittels, wobei man wenigstens eine Verbindung der Ansprüche 1 bis 9 in einer zur Verabreichung geeigneten Form, gegebenenfalls aufgenommen in einem pharmazeutisch akzeptablen Träger und/oder Exzipienten, bereitstellt.

15. Verwendung einer Verbindung nach einem der Ansprüche 1 bis 9 zur Herstellung eines pharmazeutischen Mittels zur Behandlung viraler Erkrankungen und Erkrankungen mikrobiellen Ursprungs bei Tieren, einschließlich des Menschen, oder Pflanzen.

**Revendications**

**Revendications pour les Etats contractants suivants : AT, BE, CH, DE, FR, GB, IT, LI, LU, NL, SE**

1. Composé de phosphonylméthoxy ayant la formule I ou la formule II

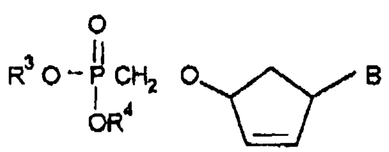

I

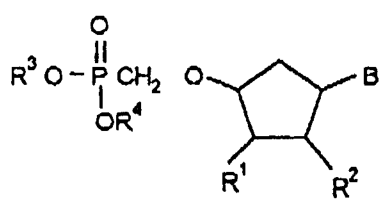

II

le groupe phosphonylméthoxy et B étant en relation stéréochimique cis,

où

$R^1$ et $R^2$

sont indépendamment hydrogène, hydroxy, chlore, brome, ou un substituant organique ayant 1 à 12 atomes de carbone et qui est choisi parmi acyloxy, alcoxy, alkylthio, amino, alkylamino et dlalkylamino,

$R^3$ et $R^4$

sont indépendamment hydrogène, ou des substituants organiques ayant 1 à 12 atomes de carbone et choisis parmi alkyle, alkényle, aryle, et aralkyle,

B est un groupe hétérocyclique ayant au moins un hétéroatome d'azote et jusqu'à trois hétéroatomes additionnels choisis parmi azote, oxygène et soufre, ledit groupe hétérocyclique étant attaché par son hétéroatome d'azote,

et leurs sels d'addition d'acide, de métal et d'amine pharmaceutiquement acceptables.

2. Composé de la revendication 1 où B est un groupe hétérocyclique choisi parmi la pyrimidine, l'azapurine, la triazine, la déazapurine, la pyridine, et le triazole, ledit groupe hétérocyclique étant substitué avec 1 à 3 substituants indépendamment choiois parmi oxo, hydroxy, amino, fluoro, chloro, bromo, iodo, alkyle ayant 1 à 3 atomes de carbone, alkényle ayant 2 à 3 atomes de carbone, haloalkényle ayant 2 à 3 atomes de carbone, alcoxy ayant 1 à 3 atomes de carbone et alkylthiol ayant 1 à 3 atomes de carbone.

3. Compose de la revendication 2 où B est un groupe hétérocyclique choisi parmi la purine monosubstituée ou la purine disubstituée.

4. Composé de la revendication 2 où B est un groupe hétérocycllque choisi parmi la pyrimidine monosubstituée, la pyrimidine disubstituée, ou la pyrimidine trisubstituée.

5. Composé de la revendication 1 ayant la configuration 1$\beta$, 4$\beta$.

6. Composés de la revendication 1 : 1-[(1$\beta$, 4$\beta$)-4-(dihydroxyphosphonyl)méthoxycyclopent-2-én-1-yl]-thymine,
   1-[(1$\beta$, 4$\beta$)-4-(dihydroxyphosphonyl)méthoxycyclopent-2-én-1-yl]cytosine,
   [(1$\beta$, 4$\beta$)-4-(diethoxyphosphonyl)méthoxycyclopent-2-én-1-yl]adénine,
   9-[(1$\beta$, 4$\beta$)-4-(éthoxyhydroxyphosphonyl)méthoxycyclopent-2-én-1-yl]adénine,
   9-[(1$\beta$, 4$\beta$)-4-(dihydroxyphosphonyl)méthoxycyclopent-2-én-1-yl]adénine,
   9-[(1$\beta$, 4$\beta$)-4-(diéthoxyphosphonyl)méthoxycyclopentan-1-yl]adénine,
   9-[(1$\beta$, 4$\beta$)-4-(dihydroxyphosphonyl)méthoxycyclopentan-1-yl]adénine

7. Composé de la revendication 1 ayant pour formule

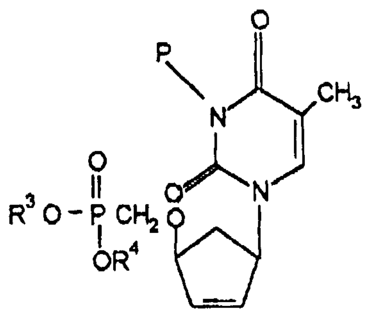

où $R^3$ et $R^4$ ont las mêmes significations que celles données à la revendication 1 et P est hydrogène ou un groupe amino protecteur.

8. Composé selon la revendication 1 ayant pour formule

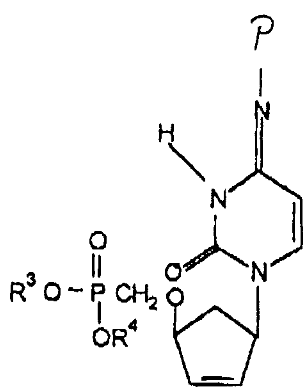

où R3 et R4 ont les mêmes définitions qu'à la revendication 1 et P est hydrogène ou un groupe amino protecteur.

9. Composé selon la revendication 1 ayant pour formule

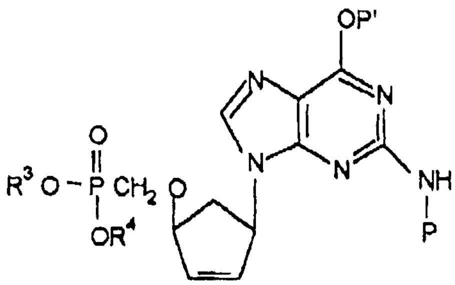

où $R^3$ et $R^4$ ont les mêmes définitions qu'à la revendication 1 et P et P' sont hydrogène ou respectivement des groupes amino ou hydroxy protecteurs.

10. Procédé de préparation d'un composé de la revendication 1 ayant la Formule I qui consiste à éthérifier un dérivé de cyclopenténol de Formule IV

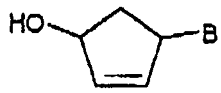

IV

avec un agent de phosphonylméthylation de Formule V

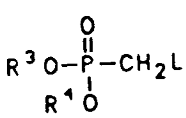

V

où à aminer un dérivé de phosphonylméthoxy cyclopenténol de Formule VI

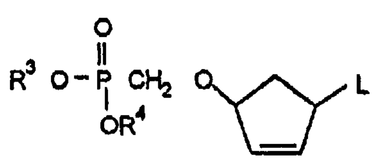

VI

avec un hétérocycle d'azote de formule BH, où $R^3$ et $R^4$ sont des substituants organiques définis à la revendication 1, B est un groupe hétérocyclique tel que défini à la revendication 1 et le groupe L dans les Formules V et VI est un groupe partant.

11. Procédé de préparation d'un composé de la revendication 1 ayant la Formule II où $R^1$ et $R^2$ sont hydrogène, qui comprend la mise en contact d'un composé de la revendication 1 ayant la Formule I avec de l'hydrogène en présence d'un catalyseur dans des conditions de réaction appropriées à la réduction d'un cyclopentène en cyclopentane.

12. Procédé de production d'un monoester d'acide phosphonique de la revendication 1 ayant la Formule I ou la Formule II ou l'un de $R^3$ et $R^4$ est hydrogène et l'autre est un substituant organique, qui comprend la mise en contact d'un composé de Formule I ou de Formule II, où $R^3$ et $R^4$ sont des substituants organiques, avec de l'eau en présence d'une base d'un métal alcalin, à température

ambiante.

**13.** Procédé de préparation d'un acide phosphonique de la revendication 1 ayant la Formule I ou la Formule II où chacun de $R^3$ et $R^4$ est hydrogène, qui comprend la mise en contact d'un composé ayant la Formule I ou la Formule II, ou l'un ou les deux de $R^3$ et $R^4$ est un substituant organique, avec du bromure de triméthylsilyle en conditions appropriées de réactions.

**14.** Composition pharmaceutique comprenant au moins un composé selon l'une quelconque des revendication 1 à 9, facultativement en association avec des supports et/ou excipients pharmaceutiquement acceptables.

**15.** Procédé de préparation d'une composition pharmaceutique selon la revendication 14 qui consiste à prévoir au moins un composé des revendications 1 à 9 sous une forme appropriée à une administration, facultativement incorporé dans un support ou excipient pharmaceutiquement acceptable.

**16.** Utilisation d'un composé selon l'une quelconque des revendications 1 à 9 pour la préparation d'une composition pharmaceutique pour le traitement de maladies virales et de maladies d'origine microbienne chez les animaux, y compris l'homme, ou les plantes.

**Revendications pour l'Etat contractant suivant : ES**

**1.** Procédé de préparation d'un phosphonylméthoxycyclopentène ayant la Formule I

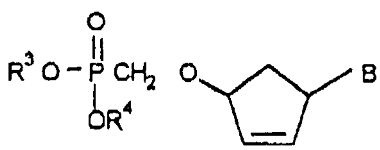

I

le groupe phosphonylméthoxy et B étant en relation stéréochimique cis,

où

$R^3$ et $R^4$

sont indépendamment hydrogène, ou des substituants organiques ayant 1 à 12 atomes de carbone et choisis parmi alkyle, alkényle, aryle, et aralkyle,

B est un groupe hetérocyclique ayant au moins un hétéroatome d'azote et jusqu'à trois hétéroatomes additionnels choisis parmi azote, oxygène et soufre, ledit groupe hétérocyclique étant attaché par son hétéroatome d'azote,

et les sels d'addition d'acide, de métal et d'amine pharmaceutiquement acceptables, qui consiste à éthérifier un dérivé de cyclopenténol de Formule IV

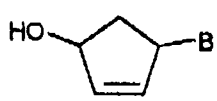

avec un agent de phosphonylméthylation de Formule V

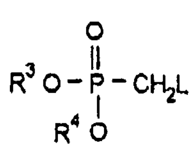

V

ou à aminer un dérivé de phosphonylméthoxy cyclopenténol de Formule VI

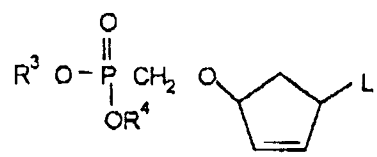

VI

avec un hétérocycle d'azote de la formule BH, où $R^3$, $R^4$ et B sont tels que définis ci-dessus et le groupe L dans les Formules V et VI est d'un groupe partant ou

à mettre en contact un composé de formule I où $R^3$ et $R^4$ sont des substituants organiques avec de l'eau, en présence d'une base d'un métal alcalin, à température ambiante pour obtenir un composé de la formule I où l'un de $R^3$ et $R^4$ est hydrogène et l'autre est un substituant d'ester phosphonique organique, ou

à mettre en contact un composé ayant la Formule I ou l'un ou les deux de $R^3$ et $R^4$ est un substituant organique, avec du bromure de triméthylsilyle en conditions appropriées de réaction pour obtenir un composé de Formule I où $R^3$ et $R^4$ sont hydrogène.

**2.** Procédé de préparation d'un phosphonylméthoxycyclopentène ayant la Formule II

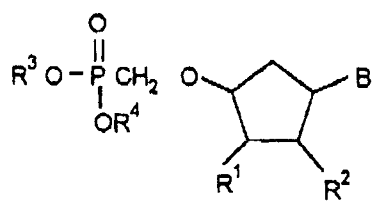

II

le groupe phosphonylméthoxy et B étant en relation stéréochimique cis,

où

$R^1$ et $R^2$

sont indépendamment hydrogène, hydroxy, chlore, brome, ou un substituant organique ayant 1 à 12 atomes de carbone et qui est choisi parmi acyloxy, alcoxy, alkylthio, amino, alkylamino et dialkylamino,

$R^3$ et $R^4$

sont indépendamment hydrogène, ou des substituants organiques ayant 1 à 12 atomes de carbone et choisis parmi alkyle, alkényle, aryle, et aralkyle,

B est un groupe hétérocyclique ayant au moins un hétéroatome d'azote et jusqu'à trois hétéroatomes additionnels choisis parmi azote, oxygène et soufre, ledit groupe hétérocyclique étant attaché par son hétéroatome d'azote,

et leurs sels d'addition d'acide, de métal et d'amine pharmaceutiquement acceptables,

qui consiste à

mettre un composé de Formule I tel que défini à la revendication 1 en contact avec de l'hydrogène en présence d'un catalyseur dans des conditions de réaction appropriées à la réduction d'un cyclopentène en un cyclopentane pour obtenir un composé de Formule II où $R^1$ et $R^2$ sont hydrogène, ou

mettre en contact un composé de Formule II où $R^3$ et $R^4$ sont des substituants organiques avec de l'eau en présence d'une base d'un métal alcalin à température ambiante pour obtenir un composé de Formule II où l'un de $R^3$ et $R^4$ est hydrogène et l'autre est un substituant organique ou

mettre en contact un composé de Formule II où l'un ou les deux de $R^3$ et $R^4$ est un substituant organique, avec du bromure de triméthylsilyle en conditions appropriées de réaction pour obtenir un composé de formule II où $R^3$ et $R^4$ sont hydrogène.

**3.** Procédé de la revendication 1 ou 2 où B est un groupe hétérocyclique choisi parmi la purine, la pyrimidine, azapurine, triazine, déazapurine, pyridine, et le triazole, ledit groupe hétérocyclique étant substitué avec 1 à 3 substituants indépendamment choisis parmi oxo, hydroxy, amino, fluoro, chloro, bromo, iodo, alkyle ayant 1 à 3 atomes de carbone, alkényle ayant 2 à 3 atomes de carbone, haloalkényl ayant 2 à 3 atomes de carbone, alcoxy ayant 1 à 3 atomes de carbone et alkylthiol ayant 1 à 3 atomes de carbone.

**4.** Procédé de la revendication 3 où B est un groupe hétérocyclique choisi parmi la purine monosubstituée, ou la purine disubstituée.

**5.** Procédé de la revendication 3 où B est un groupe hétérocyclique choisi parmi la pyrimidine monosubstituée, la pyrimidine disubstituée, ou la pyrimidine trisubstituée.

**6.** Procédé de la revendication 1 ou 2 ayant la configuration 1$\beta$, 4$\beta$.

**7.** Procédé de la revendication 2 pour la préparation des composés suivants :
1-[(1$\beta$, 4$\beta$)-(dihydroxyphosphonyl)méthoxycyclopent-2-én-1-yl]thymine,
1-[(1$\beta$, 4$\beta$)-4-(dihydroxyphosphonyl)méthoxycyclopent-2-én-1-yl]cytosine,
[(1$\beta$, 4$\beta$)-4-(diéthoxyphosphonyl)méthoxycyclopent-2-én-1-yl]adenine,
9-[(1$\beta$, 4$\beta$)-4-(éthoxyhydroxyphosphonyl)méthoxycyclopent-2-én-1-yl]adénine,
9-[(1$\beta$, 4$\beta$)-4-(dihydroxyphosphonyl)méthoxycyclopent-2-én-1-yl]adénine,
9-[(1$\beta$, 4$\beta$)-4-(diéthoxyphosphonyl)méthoxycyclopentan-1-yl]adénine,
9-[(1$\beta$, 4$\beta$)-4-(dihydroxyphosphonyl)méthoxycyclopentan-1-yl]adénine.

**8.** Procédé de la revendication 1 pour la préparation d'un compose ayant pour formule

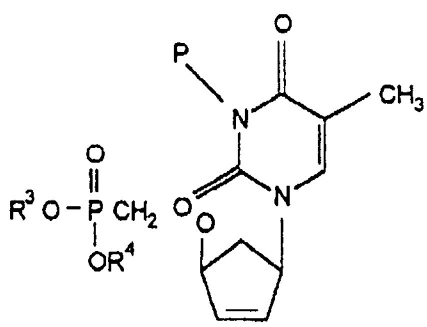

où $R^3$ et $R^4$ ont les mêmes significations que celles données à la revendication 1 et P est hydrogène ou un groupe amino protecteur.

**9.** Procédé de la revendication 1 pour la préparation d'un composé ayant pour formule

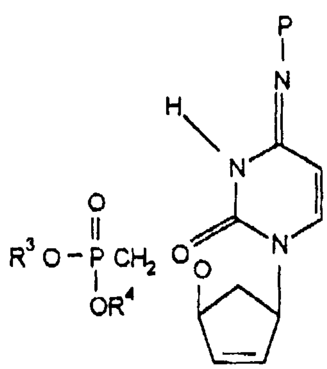

où $R^3$ et $R^4$ ont les mêmes définitions qu'à la revendication 1 et P est hydrogène ou un groupe amino protecteur.

**10.** Procédé de la revendication 1 pour la préparation d'un composé ayant pour formule

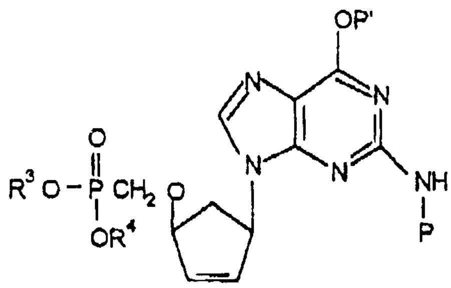

où $R^3$ et $R^4$ ont les mêmes définitions qu'à la revendication 1, et P et P' sont respectivement des groupes amino et hydroxy protecteurs.

**11.** Procédé de préparation d'une composition pharmaceutique qui consiste à prévoir au moins un composé tel que défini selon l'une des revendications 1 à 10 sous une forme appropriée pour une administration, facultativement incorporée dans un support et/ou excipient pharmaceutiquement acceptable.

**Revendications pour l'Etat contractant suivant : GR**

**1.** Composé de phosphonylméthoxy ayant la formule I ou la formule II

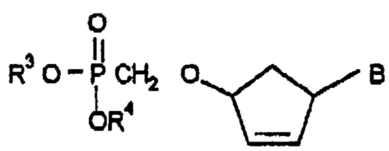

I

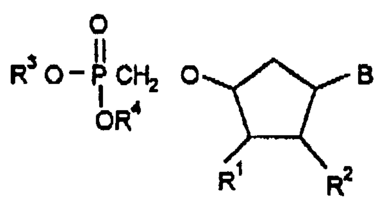

II

le groupe phosphonylméthoxy et B étant en relation stéréochimique cis,

où

$R^1$ et $R^2$

sont indépendamment hydrogène, hydroxy, chlore, brome, ou un substituant organique ayant 1 à 12 atomes de carbone et qui est choisi parmi acyloxy, alcoxy, alkylthio, amino, alkylamino et dialkylamino,

$R^3$ et $R^4$

sont indépendamment hydrogène, ou des substituants organiques ayant 1 à 12 atomes de carbone et choisis parmi alkyle, alkényle, aryle, et aralkyle,

B est un groupe hétérocyclique ayant au moins un hétéroatome d'azote et jusqu'à trois hétéroatomes additionnels choisis parmi azote, oxygène et soufre, ledit groupe hétérocyclique étant attaché par son hétéroatome d'azote,

et leurs sels d'addition d'acide, de métal et d'amine pharmaceutiquement acceptables.

**2.** Composé de la revendication 1 où B est un groupe hétérocyclique choisi parmi la pyrimidine, l'azapurine, la triazine, la déazapurine, la pyridine, et le triazole, ledit groupe hétérocyclique étant substitue avec 1 à 3 substituants indépendamment choisis parmi oxo, hydroxy, amino, fluoro, chloro, bromo, iodo, alkyle ayant 1 à 3 atomes de carbone, alkényle ayant 2 à 3 atomes de carbone, haloalkényle ayant 2 à 3 atomes de carbone, alcoxy ayant 1 à 3 atomes de carbone et alkylthiol ayant 1 à 3 atomes de carbone.

3. Composé de la revendication 2 où B est un groupe hétérocyclique choisi parmi la purine monosubstituée ou la purine disubstituée.

4. Composé de la revendication 2 où B est un groupe hétérocyclique choisi parmi la pyrimidine monosubstituée, la pyrimidine disubstituée, ou la pyrimidine trisubstituée.

5. Composé de la revendication 1 ayant la configuration 1$\beta$, 4$\beta$.

6. Composés de la revendication 1 : 1-[(1$\beta$, 4$\beta$)-4-(dihydroxyphosphonyl)méthoxycyclopent-2-én-1-yl]-thymine,
1-[(1$\beta$, 4$\beta$)-4-(dihydroxyphosphonyl)méthoxycyclopent-2-én-1-yl]cytosine,
[(1$\beta$, 4$\beta$)-4-(diethoxyphosphonyl)méthoxycyclopent-2-én-1-yl]adénine,
9-[(1$\beta$, 4$\beta$)-4-(éthoxyhydroxyphosphonyl)méthoxycyclopent-2-én-1-yl]adénine,
9-[(1$\beta$, 4$\beta$)-4-(dihydroxyphosphonyl)méthoxycyclopent-2-én-1-yl]adénine,
9-[(1$\beta$, 4$\beta$)-4-(diéthoxyphosphonyl)méthoxycyclopentan-1-yl]adénine,
9-[(1$\beta$, 4$\beta$)-4-(dihydroxyphosphonyl)méthoxycyclopentan-1-yl]adénine.

7. Composé de la revendication 1 ayant pour formule

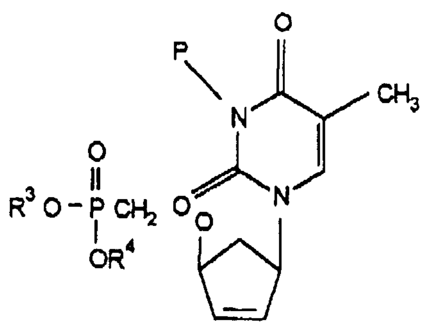

où $R^3$ et $R^4$ ont les mêmes significations que celles données à la revendication 1 et P est hydrogène ou un groupe amino protecteur.

8. Composé selon la revendication 1 ayant pour formule

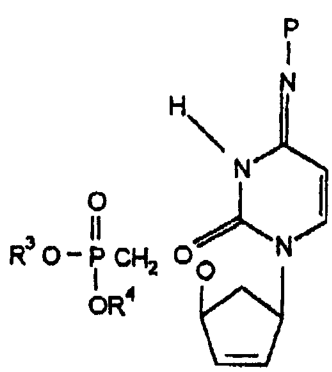

où R3 et R4 ont les mêmes définitions qu'à la revendication 1 et P est hydrogène ou un groupe amino protecteur.

**9.** Composé selon la revendication 1 ayant pour formule

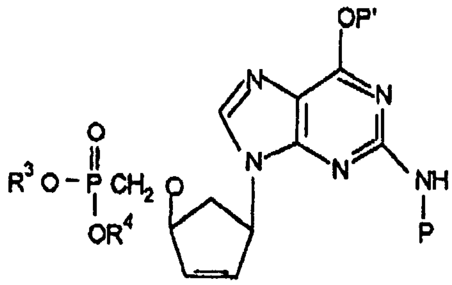

où $R^3$ et $R^4$ ont les mêmes définitions qu'à la revendication 1 et P et P' sont hydrogène ou respectivement des groupes amino ou hydroxy protecteurs.

**10.** Procédé de préparation d'un composé de la revendication 1 ayant la Formule I qui consiste à éthérifier un dérivé de cyclopenténol de Formule IV

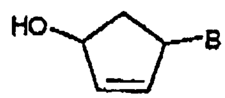

IV

avec un agent de phosphonylméthylation de Formule V

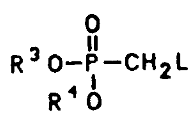

V

où à aminer un dérivé de phosphonylméthoxy cyclopenténol de Formule VI

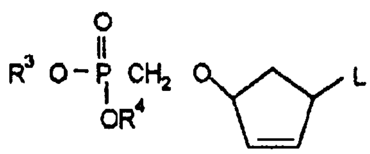

VI

avec un hétérocycle d'azote de formule BH, où $R^3$ et $R^4$ sont des substituants organiques définis à la revendication 1, B est un groupe hétérocyclique tel que défini à la revendication 1 et le groupe L dans les Formules V et VI est un groupe partant.

**11.** Procédé de préparation d'un composé de la revendication 1 ayant la Formule II où $R^1$ et $R^2$ sont hydrogène, qui comprend la mise en contact d'un composé de la revendication 1 ayant la Formule I avec de l'hydrogène en présence d'un catalyseur dans des conditions de réaction appropriées à la réduction d'un cyclopentène en cyclopentane.

**12.** Procédé de production d'un monoester d'acide phosphonique de la revendication 1 ayant la Formule I ou la Formule II ou l'un de $R^3$ et $R^4$ est hydrogène et l'autre est un substituant organique, qui comprend la mise en contact d'un composé de Formule I ou de Formule II, où $R^3$ et $R^4$ sont des substituants organiques, avec de l'eau en présence d'une base d'un métal alcalin, à température ambiante.

13. Procédé de préparation d'un acide phosphonique de la revendication 1 ayant la Formule I ou la Formule II où chacun de $R^3$ et $R^4$ est hydrogène, qui comprend la mise en contact d'un composé ayant le Formule I ou la Formule II, ou l'un ou les deux de $R^3$ et $R^4$ est un subsituant organique, avec du bromure de triméthylsilyle en conditions appropriées de réactions.

14. Procédé de préparation d'une composition pharmaceutique qui consiste à prévoir au moins un composé des revendications 1 à 9 sous une forme appropriée à une administration facultativement incorporée dans un support et/ou excipient pharmaceutiquement acceptable.

15. Utilisation d'un composé selon l'une quelconque des revendications 1 à 9 pour la préparation d'une composition pharmaceutique pour le traitement de maladies virales et de maladies microbiennes chez les animaux, comprenant l'homme, ou les plantes.